# FASCICULE DE BREVET EUROPEEN

(11) **EP 2 678 355 B1**
(45) Date de publication et mention de la délivrance du brevet: **17.08.2016**
(21) Numéro de dépôt: 12705692.7
(22) Date de dépôt: 24.02.2012
(51) Int. Cl.: C07K 14/54, C07K 16/24

(54) **NOUVEAUX FRAGMENTS D'IL-33 SUPERACTIFS ET LEURS UTILISATIONS**
SUPERAKTIVE NEUE IL-33-FRAGMENTE UND IHRE VERWENDUNG
NOVEL SUPERACTIVE IL-33 FRAGMENTS, AND USES THEREOF

(30) Priorité: 24.02.2011 FR 1151498
(43) Date de publication de la demande: 01.01.2014
(73) Titulaire: Centre National de la Recherche Scientifique (CNRS), 75016 Paris (FR)
(72) Inventeur: GIRARD, Jean-Philippe, 31320 Rebigue (FR); CAYROL-GIRARD, Corinne, F-31320 Rebigue (FR); LEFRANCAIS, Emma, 40000 Mont de Marsan (FR)
(74) Mandataire: Regimbeau
(86) Numéro de dépôt international: PCT/EP2012/053199
(87) Numéro de publication internationale: WO 2012/113927

(56) Documents cités:
- WO-A1-2008/132709
- CAYROL CORINNE ET AL: "The IL-1-like cytokine IL-33 is inactivated after maturation by caspase-1.", PROCEEDINGS OF THE NATIONAL ACADEMY OF SCIENCES OF THE UNITED STATES OF AMERICA 2 JUN 2009 LNKD- PUBMED:19439663, vol. 106, no. 22, 2 juin 2009 (2009-06-02) , pages 9021-9026, XP002658772, ISSN: 1091-6490
- LÜTHI ALEXANDER U ET AL: "Suppression of interleukin-33 bioactivity through proteolysis by apoptotic caspases.", IMMUNITY 17 JUL 2009 LNKD- PUBMED:19559631, vol. 31, no. 1, 17 juillet 2009 (2009-07-17), pages 84-98, XP002658773, ISSN: 1097-4180
- SCHMITZ JOCHEN ET AL: "IL-33, an interleukin-1-like cytokine that signals via the IL-1 receptor-related protein ST2 and induces T helper type 2-associated cytokines.", IMMUNITY NOV 2005 LNKD- PUBMED:16286016, vol. 23, no. 5, novembre 2005 (2005-11), pages 479-490, XP002658774, ISSN: 1074-7613
- LIEW FOO Y ET AL: "Disease-associated functions of IL-33: the new kid in the IL-1 family.", NATURE REVIEWS. IMMUNOLOGY FEB 2010 LNKD- PUBMED:20081870, vol. 10, no. 2, février 2010 (2010-02), pages 103-110, XP002658775, ISSN: 1474-1741
- KUROWSKA-STOLARSKA M ET AL: "Interleukin-33: a novel mediator with a role in distinct disease pathologies.", JOURNAL OF INTERNAL MEDICINE JAN 2011 LNKD- DOI:10.1111/J.1365-2796.2010.02316.X PUBMED:21158975, vol. 269, no. 1, janvier 2011 (2011-01), pages 29-35, XP002658776, ISSN: 1365-2796

### SEQUENCE LISTING

<110> CENTRE NATIONAL DE LA RECHERCHE SCIENTIFIQUE (CNRS)
<120> NOUVEAUX FRAGMENTS D'IL-33 SUPERACTIFS ET LEURS UTILISATIONS
<130> 360405D29350
<150> FR 1151498
   <151> 2011-02-24
<160> 16
<170> PatentIn version 3.5
<210> 1
   <211> 270
   <212> PRT
   <213> homo sapiens
<400> 1
   Met Lys Pro Lys Met Lys Tyr Ser Thr Asn Lys Ile Ser Thr Ala Lys
   1               5                   10                  15
   Trp Lys Asn Thr Ala Ser Lys Ala Leu Cys Phe Lys Leu Gly Lys Ser
               20                  25                  30
   Gln Gln Lys Ala Lys Glu Val Cys Pro Met Tyr Phe Met Lys Leu Arg
           35                  40                  45
   Ser Gly Leu Met Ile Lys Lys Glu Ala Cys Tyr Phe Arg Arg Glu Thr
       50                  55                  60
   Thr Lys Arg Pro Ser Leu Lys Thr Gly Arg Lys His Lys Arg His Leu
   65                  70                  75                  80
   Val Leu Ala Ala Cys Gln Gln Gln Ser Thr Val Glu Cys Phe Ala Phe
                   85                  90                  95
   Gly Ile Ser Gly Val Gln Lys Tyr Thr Arg Ala Leu His Asp Ser Ser
               100                 105                 110
   Ile Thr Gly Ile Ser Pro Ile Thr Glu Tyr Leu Ala Ser Leu Ser Thr
           115                 120                 125
   Tyr Asn Asp Gln Ser Ile Thr Phe Ala Leu Glu Asp Glu Ser Tyr Glu
       130                 135                 140
   Ile Tyr Val Glu Asp Leu Lys Lys Asp Glu Lys Lys Asp Lys Val Leu
   145                 150                 155                 160
   Leu Ser Tyr Tyr Glu Ser Gln His Pro Ser Asn Glu Ser Gly Asp Gly
                   165                 170                 175
   Val Asp Gly Lys Met Leu Met Val Thr Leu Ser Pro Thr Lys Asp Phe
               180                 185                 190
   Trp Leu His Ala Asn Asn Lys Glu His Ser Val Glu Leu His Lys Cys
           195                 200                 205
   Glu Lys Pro Leu Pro Asp Gln Ala Phe Phe Val Leu His Asn Met His
       210                 215                 220
   Ser Asn Cys Val Ser Phe Glu Cys Lys Thr Asp Pro Gly Val Phe Ile
   225                 230                 235                 240
   Gly Val Lys Asp Asn His Leu Ala Leu Ile Lys Val Asp Ser Ser Glu
                   245                 250                 255
   Asn Leu Cys Thr Glu Asn Ile Leu Phe Lys Leu Ser Glu Thr
               260                 265                 270
<210> 2
   <211> 176
   <212> PRT
   <213> homo sapiens
<400> 2
   Ala Phe Gly Ile Ser Gly Val Gln Lys Tyr Thr Arg Ala Leu His Asp
   1               5                   10                  15
   Ser Ser Ile Thr Gly Ile Ser Pro Ile Thr Glu Tyr Leu Ala Ser Leu
               20                  25                  30
   Ser Thr Tyr Asn Asp Gln Ser Ile Thr Phe Ala Leu Glu Asp Glu Ser
           35                  40                  45
   Tyr Glu Ile Tyr Val Glu Asp Leu Lys Lys Asp Glu Lys Lys Asp Lys
       50                  55                  60
   Val Leu Leu Ser Tyr Tyr Glu Ser Gln His Pro Ser Asn Glu Ser Gly
   65                  70                  75                  80
   Asp Gly Val Asp Gly Lys Met Leu Met Val Thr Leu Ser Pro Thr Lys
                   85                  90                  95
   Asp Phe Trp Leu His Ala Asn Asn Lys Glu His Ser Val Glu Leu His
               100                 105                 110
   Lys Cys Glu Lys Pro Leu Pro Asp Gln Ala Phe Phe Val Leu His Asn
           115                 120                 125
   Met His Ser Asn Cys Val Ser Phe Glu Cys Lys Thr Asp Pro Gly Val
       130                 135                 140
   Phe Ile Gly Val Lys Asp Asn His Leu Ala Leu Ile Lys Val Asp Ser
   145                 150                 155                 160
   Ser Glu Asn Leu Cys Thr Glu Asn Ile Leu Phe Lys Leu Ser Glu Thr
                   165                 170                 175
<210> 3
   <211> 172
   <212> PRT
   <213> homo sapiens
<400> 3
   Ser Gly Val Gln Lys Tyr Thr Arg Ala Leu His Asp Ser Ser Ile Thr
   1               5                   10                  15
   Gly Ile Ser Pro Ile Thr Glu Tyr Leu Ala Ser Leu Ser Thr Tyr Asn
               20                  25                  30
   Asp Gln Ser Ile Thr Phe Ala Leu Glu Asp Glu Ser Tyr Glu Ile Tyr
           35                  40                  45
   Val Glu Asp Leu Lys Lys Asp Glu Lys Lys Asp Lys Val Leu Leu Ser
       50                  55                  60
   Tyr Tyr Glu Ser Gln His Pro Ser Asn Glu Ser Gly Asp Gly Val Asp
   65                  70                  75                  80
   Gly Lys Met Leu Met Val Thr Leu Ser Pro Thr Lys Asp Phe Trp Leu
                   85                  90                  95
   His Ala Asn Asn Lys Glu His Ser Val Glu Leu His Lys Cys Glu Lys
               100                 105                 110
   Pro Leu Pro Asp Gln Ala Phe Phe Val Leu His Asn Met His Ser Asn
           115                 120                 125
   Cys Val Ser Phe Glu Cys Lys Thr Asp Pro Gly Val Phe Ile Gly Val
       130                 135                 140
   Lys Asp Asn His Leu Ala Leu Ile Lys Val Asp Ser Ser Glu Asn Leu
   145                 150                 155                 160
   Cys Thr Glu Asn Ile Leu Phe Lys Leu Ser Glu Thr
                   165                 170
<210> 4
   <211> 162
   <212> PRT
   <213> homo sapiens
<400> 4
   His Asp Ser Ser Ile Thr Gly Ile Ser Pro Ile Thr Glu Tyr Leu Ala
   1               5                   10                  15
   Ser Leu Ser Thr Tyr Asn Asp Gln Ser Ile Thr Phe Ala Leu Glu Asp
               20                  25                  30
   Glu Ser Tyr Glu Ile Tyr Val Glu Asp Leu Lys Lys Asp Glu Lys Lys
           35                  40                  45
   Asp Lys Val Leu Leu Ser Tyr Tyr Glu Ser Gln His Pro Ser Asn Glu
       50                  55                  60
   Ser Gly Asp Gly Val Asp Gly Lys Met Leu Met Val Thr Leu Ser Pro
   65                  70                  75                  80
   Thr Lys Asp Phe Trp Leu His Ala Asn Asn Lys Glu His Ser Val Glu
                   85                  90                  95
   Leu His Lys Cys Glu Lys Pro Leu Pro Asp Gln Ala Phe Phe Val Leu
               100                 105                 110
   His Asn Met His Ser Asn Cys Val Ser Phe Glu Cys Lys Thr Asp Pro
           115                 120                 125
   Gly Val Phe Ile Gly Val Lys Asp Asn His Leu Ala Leu Ile Lys Val
       130                 135                 140
   Asp Ser Ser Glu Asn Leu Cys Thr Glu Asn Ile Leu Phe Lys Leu Ser
   145                 150                 155                 160
   Glu Thr
<210> 5
   <211> 159
   <212> PRT
   <213> Artificial Sequence
<220>
   <223> Forme tronquée de IL-33 humaine Hs IL33 acides aminés 112-270
<400> 5
   Ser Ile Thr Gly Ile Ser Pro Ile Thr Glu Tyr Leu Ala Ser Leu Ser
   1               5                   10                  15
   Thr Tyr Asn Asp Gln Ser Ile Thr Phe Ala Leu Glu Asp Glu Ser Tyr
               20                  25                  30
   Glu Ile Tyr Val Glu Asp Leu Lys Lys Asp Glu Lys Lys Asp Lys Val
           35                  40                  45
   Leu Leu Ser Tyr Tyr Glu Ser Gln His Pro Ser Asn Glu Ser Gly Asp
       50                  55                  60
   Gly Val Asp Gly Lys Met Leu Met Val Thr Leu Ser Pro Thr Lys Asp
   65                  70                  75                  80
   Phe Trp Leu His Ala Asn Asn Lys Glu His Ser Val Glu Leu His Lys
                   85                  90                  95
   Cys Glu Lys Pro Leu Pro Asp Gln Ala Phe Phe Val Leu His Asn Met
               100                 105                 110
   His Ser Asn Cys Val Ser Phe Glu Cys Lys Thr Asp Pro Gly Val Phe
           115                 120                 125
   Ile Gly Val Lys Asp Asn His Leu Ala Leu Ile Lys Val Asp Ser Ser
       130                 135                 140
   Glu Asn Leu Cys Thr Glu Asn Ile Leu Phe Lys Leu Ser Glu Thr
   145                 150                 155
<210> 6
   <211> 255
   <212> PRT
   <213> homo sapiens
<400> 6
   Met Gln Pro Leu Leu Leu Leu Leu Ala Phe Leu Leu Pro Thr Gly Ala
   1               5                   10                  15
   Glu Ala Gly Glu Ile Ile Gly Gly Arg Glu Ser Arg Pro His Ser Arg
               20                  25                  30
   Pro Tyr Met Ala Tyr Leu Gln Ile Gln Ser Pro Ala Gly Gln Ser Arg
           35                  40                  45
   Cys Gly Gly Phe Leu Val Arg Glu Asp Phe Val Leu Thr Ala Ala His
       50                  55                  60
   Cys Trp Gly Ser Asn Ile Asn Val Thr Leu Gly Ala His Asn Ile Gln
   65                  70                  75                  80
   Arg Arg Glu Asn Thr Gln Gln His Ile Thr Ala Arg Arg Ala Ile Arg
                   85                  90                  95
   His Pro Gln Tyr Asn Gln Arg Thr Ile Gln Asn Asp Ile Met Leu Leu
               100                 105                 110
   Gln Leu Ser Arg Arg Val Arg Arg Asn Arg Asn Val Asn Pro Val Ala
           115                 120                 125
   Leu Pro Arg Ala Gln Glu Gly Leu Arg Pro Gly Thr Leu Cys Thr Val
       130                 135                 140
   Ala Gly Trp Gly Arg Val Ser Met Arg Arg Gly Thr Asp Thr Leu Arg
   145                 150                 155                 160
   Glu Val Gln Leu Arg Val Gln Arg Asp Arg Gln Cys Leu Arg Ile Phe
                   165                 170                 175
   Gly Ser Tyr Asp Pro Arg Arg Gln Ile Cys Val Gly Asp Arg Arg Glu
               180                 185                 190
   Arg Lys Ala Ala Phe Lys Gly Asp Ser Gly Gly Pro Leu Leu Cys Asn
           195                 200                 205
   Asn Val Ala His Gly Ile Val Ser Tyr Gly Lys Ser Ser Gly Val Pro
       210                 215                 220
   Pro Glu Val Phe Thr Arg Val Ser Ser Phe Leu Pro Trp Ile Arg Thr
   225                 230                 235                 240
   Thr Met Arg Ser Phe Lys Leu Leu Asp Gln Met Glu Thr Pro Leu
                   245                 250                 255
<210> 7
   <211> 256
   <212> PRT
   <213> homo sapiens
<400> 7
   Met Ala His Arg Pro Pro Ser Pro Ala Leu Ala Ser Val Leu Leu Ala
   1               5                   10                  15
   Leu Leu Leu Ser Gly Ala Ala Arg Ala Ala Glu Ile Val Gly Gly His
               20                  25                  30
   Glu Ala Gln Pro His Ser Arg Pro Tyr Met Ala Ser Leu Gln Met Arg
           35                  40                  45
   Gly Asn Pro Gly Ser His Phe Cys Gly Gly Thr Leu Ile His Pro Ser
       50                  55                  60
   Phe Val Leu Thr Ala Ala His Cys Leu Arg Asp Ile Pro Gln Arg Leu
   65                  70                  75                  80
   Val Asn Val Val Leu Gly Ala His Asn Val Arg Thr Gln Glu Pro Thr
                   85                  90                  95
   Gln Gln His Phe Ser Val Ala Gln Val Phe Leu Asn Asn Tyr Asp Ala
               100                 105                 110
   Glu Asn Lys Leu Asn Asp Val Leu Leu Ile Gln Leu Ser Ser Pro Ala
           115                 120                 125
   Asn Leu Ser Ala Ser Val Ala Thr Val Gln Leu Pro Gln Gln Asp Gln
       130                 135                 140
   Pro Val Pro His Gly Thr Gln Cys Leu Ala Met Gly Trp Gly Arg Val
   145                 150                 155                 160
   Gly Ala His Asp Pro Pro Ala Gln Val Leu Gln Glu Leu Asn Val Thr
                   165                 170                 175
   Val Val Thr Phe Phe Cys Arg Pro His Asn Ile Cys Thr Phe Val Pro
               180                 185                 190
   Arg Arg Lys Ala Gly Ile Cys Phe Gly Asp Ser Gly Gly Pro Leu Ile
           195                 200                 205
   Cys Asp Gly Ile Ile Gln Gly Ile Asp Ser Phe Val Ile Trp Gly Cys
       210                 215                 220
   Ala Thr Arg Leu Phe Pro Asp Phe Phe Thr Arg Val Ala Leu Tyr Val
   225                 230                 235                 240
   Asp Trp Ile Arg Ser Thr Leu Arg Arg Val Glu Ala Lys Gly Arg Pro
                   245                 250                 255
<210> 8
   <211> 267
   <212> PRT
   <213> homo sapiens
<400> 8
<210> 9
   <211> 528
   <212> DNA
   <213> homo sapiens
<400> 9
   gcctttggta tatcaggggt ccagaaatat actagagcac ttcatgattc aagtatcaca 60
   ggaatttcac ctattacaga gtatcttgct tctctaagca catacaatga tcaatccatt 120
   acttttgctt tggaggatga aagttatgag atatatgttg aagacttgaa aaaagatgaa 180
   aagaaagata aggtgttact gagttactat gagtctcaac acccctcaaa tgaatcaggt 240
   gacggtgttg atggtaagat gttaatggta accctgagtc ctacaaaaga cttctggttg 300
   catgccaaca acaaggaaca ctctgtggag ctccataagt gtgaaaaacc actgccagac 360
   caggccttct ttgtccttca taatatgcac tccaactgtg tttcatttga atgcaagact 420
   gatcctggag tgtttatagg tgtaaaggat aatcatcttg ctctgattaa agtagactct 480
   tctgagaatt tgtgtactga aaatatcttg tttaagctct ctgaaact 528
<210> 10
   <211> 516
   <212> DNA
   <213> homo sapiens
<400> 10
   tcaggggtcc agaaatatac tagagcactt catgattcaa gtatcacagg aatttcacct 60
   attacagagt atcttgcttc tctaagcaca tacaatgatc aatccattac ttttgctttg 120
   gaggatgaaa gttatgagat atatgttgaa gacttgaaaa aagatgaaaa gaaagataag 180
   gtgttactga gttactatga gtctcaacac ccctcaaatg aatcaggtga cggtgttgat 240
   ggtaagatgt taatggtaac cctgagtcct acaaaagact tctggttgca tgccaacaac 300
   aaggaacact ctgtggagct ccataagtgt gaaaaaccac tgccagacca ggccttcttt 360
   gtccttcata atatgcactc caactgtgtt tcatttgaat gcaagactga tcctggagtg 420
   tttataggtg taaaggataa tcatcttgct ctgattaaag tagactcttc tgagaatttg 480
   tgtactgaaa atatcttgtt taagctctct gaaact 516
<210> 11
   <211> 486
   <212> DNA
   <213> homo sapiens
<400> 11
   catgattcaa gtatcacagg aatttcacct attacagagt atcttgcttc tctaagcaca        60
   tacaatgatc aatccattac ttttgctttg gaggatgaaa gttatgagat atatgttgaa       120
   gacttgaaaa aagatgaaaa gaaagataag gtgttactga gttactatga gtctcaacac       180
   ccctcaaatg aatcaggtga cggtgttgat ggtaagatgt taatggtaac cctgagtcct       240
   acaaaagact tctggttgca tgccaacaac aaggaacact ctgtggagct ccataagtgt       300
   gaaaaaccac tgccagacca ggccttcttt gtccttcata atatgcactc caactgtgtt       360
   tcatttgaat gcaagactga tcctggagtg tttataggtg taaaggataa tcatcttgct       420
   ctgattaaag tagactcttc tgagaatttg tgtactgaaa atatcttgtt taagctctct       480
   gaaact                                                                  486
<210> 12
   <211> 556
   <212> PRT
   <213> homo sapiens
<400> 12
   Met Gly Phe Trp Ile Leu Ala Ile Leu Thr Ile Leu Met Tyr Ser Thr
   1               5                   10                  15
   Ala Ala Lys Phe Ser Lys Gln Ser Trp Gly Leu Glu Asn Glu Ala Leu
               20                  25                  30
   Ile Val Arg Cys Pro Arg Gln Gly Lys Pro Ser Tyr Thr Val Asp Trp
           35                  40                  45
   Tyr Tyr Ser Gln Thr Asn Lys Ser Ile Pro Thr Gln Glu Arg Asn Arg
       50                  55                  60
   Val Phe Ala Ser Gly Gln Leu Leu Lys Phe Leu Pro Ala Ala Val Ala
   65                  70                  75                  80
   Asp Ser Gly Ile Tyr Thr Cys Ile Val Arg Ser Pro Thr Phe Asn Arg
                   85                  90                  95
   Thr Gly Tyr Ala Asn Val Thr Ile Tyr Lys Lys Gln Ser Asp Cys Asn
               100                 105                 110
   Val Pro Asp Tyr Leu Met Tyr Ser Thr Val Ser Gly Ser Glu Lys Asn
           115                 120                 125
   Ser Lys Ile Tyr Cys Pro Thr Ile Asp Leu Tyr Asn Trp Thr Ala Pro
       130                 135                 140
   Leu Glu Trp Phe Lys Asn Cys Gln Ala Leu Gln Gly Ser Arg Tyr Arg
   145                 150                 155                 160
   Ala His Lys Ser Phe Leu Val Ile Asp Asn Val Met Thr Glu Asp Ala
                   165                 170                 175
   Gly Asp Tyr Thr Cys Lys Phe Ile His Asn Glu Asn Gly Ala Asn Tyr
               180                 185                 190
   Ser Val Thr Ala Thr Arg Ser Phe Thr Val Lys Asp Glu Gln Gly Phe
           195                 200                 205
   Ser Leu Phe Pro Val Ile Gly Ala Pro Ala Gln Asn Glu Ile Lys Glu
       210                 215                 220
   Val Glu Ile Gly Lys Asn Ala Asn Leu Thr Cys Ser Ala Cys Phe Gly
   225                 230                 235                 240
   Lys Gly Thr Gln Phe Leu Ala Ala Val Leu Trp Gln Leu Asn Gly Thr
                   245                 250                 255
   Lys Ile Thr Asp Phe Gly Glu Pro Arg Ile Gln Gln Glu Glu Gly Gln
               260                 265                 270
   Asn Gln Ser Phe Ser Asn Gly Leu Ala Cys Leu Asp Met Val Leu Arg
           275                 280                 285
   Ile Ala Asp Val Lys Glu Glu Asp Leu Leu Leu Gln Tyr Asp Cys Leu
       290                 295                 300
   Ala Leu Asn Leu His Gly Leu Arg Arg His Thr Val Arg Leu Ser Arg
   305                 310                 315                 320
   Lys Asn Pro Ile Asp His His Ser Ile Tyr Cys Ile Ile Ala Val Cys
                   325                 330                 335
   Ser Val Phe Leu Met Leu Ile Asn Val Leu Val Ile Ile Leu Lys Met
               340                 345                 350
   Phe Trp Ile Glu Ala Thr Leu Leu Trp Arg Asp Ile Ala Lys Pro Tyr
           355                 360                 365
   Lys Thr Arg Asn Asp Gly Lys Leu Tyr Asp Ala Tyr Val Val Tyr Pro
       370                 375                 380
   Arg Asn Tyr Lys Ser Ser Thr Asp Gly Ala Ser Arg Val Glu His Phe
   385                 390                 395                 400
   Val His Gln Ile Leu Pro Asp Val Leu Glu Asn Lys Cys Gly Tyr Thr
   405 410 415
   Leu Cys Ile Tyr Gly Arg Asp Met Leu Pro Gly Glu Asp Val Val Thr
   420 425 430
   Ala Val Glu Thr Asn Ile Arg Lys Ser Arg Arg His Ile Phe Ile Leu
   435 440 445
   Thr Pro Gln Ile Thr His Asn Lys Glu Phe Ala Tyr Glu Gln Glu Val
   450 455 460
   Ala Leu His Cys Ala Leu Ile Gln Asn Asp Ala Lys Val Ile Leu Ile
   465 470 475 480
   Glu Met Glu Ala Leu Ser Glu Leu Asp Met Leu Gln Ala Glu Ala Leu
   485 490 495
   Gln Asp Ser Leu Gln His Leu Met Lys Val Gln Gly Thr Ile Lys Trp
   500 505 510
   Arg Glu Asp His Ile Ala Asn Lys Arg Ser Leu Asn Ser Lys Phe Trp
   515 520 525
   Lys His Val Arg Tyr Gln Met Pro Val Pro Ser Lys Ile Pro Arg Lys
   530 535 540
   Ala Ser Ser Leu Thr Pro Leu Ala Ala Gln Lys Gln
   545 550 555
<210> 14
   <211> 5428
   <212> DNA
   <213> artificial
<220>
   <223> DNA sequence de pcDNA3.1 (+)
<400> 14
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctagc     900
   gtttaaactt aagcttggta ccgagctcgg atccactagt ccagtgtggt ggaattctgc     960
   agatatccag cacagtggcg gccgctcgag tctagagggc ccgtttaaac ccgctgatca    1020
   gcctcgactg tgccttctag ttgccagcca tctgttgttt gcccctcccc cgtgccttcc    1080
   ttgaccctgg aaggtgccac tcccactgtc ctttcctaat aaaatgagga aattgcatcg    1140
   cattgtctga gtaggtgtca ttctattctg gggggtgggg tggggcagga cagcaagggg    1200
   gaggattggg aagacaatag caggcatgct ggggatgcgg tgggctctat ggcttctgag    1260
   gcggaaagaa ccagctgggg ctctaggggg tatccccacg cgccctgtag cggcgcatta    1320
   agcgcggcgg gtgtggtggt tacgcgcagc gtgaccgcta cacttgccag cgccctagcg    1380
   cccgctcctt tcgctttctt cccttccttt ctcgccacgt tcgccggctt tccccgtcaa    1440
   gctctaaatc gggggctccc tttagggttc cgatttagtg ctttacggca cctcgacccc    1500
   aaaaaacttg attagggtga tggttcacgt agtgggccat cgccctgata gacggttttt 1560
   cgccctttga cgttggagtc cacgttcttt aatagtggac tcttgttcca aactggaaca 1620
   acactcaacc ctatctcggt ctattctttt gatttataag ggattttgcc gatttcggcc 1680
   tattggttaa aaaatgagct gatttaacaa aaatttaacg cgaattaatt ctgtggaatg 1740
   tgtgtcagtt agggtgtgga aagtccccag gctccccagc aggcagaagt atgcaaagca 1800
   tgcatctcaa ttagtcagca accaggtgtg gaaagtcccc aggctcccca gcaggcagaa 1860
   gtatgcaaag catgcatctc aattagtcag caaccatagt cccgccccta actccgccca 1920
   tcccgcccct aactccgccc agttccgccc attctccgcc ccatggctga ctaatttttt 1980
   ttatttatgc agaggccgag gccgcctctg cctctgagct attccagaag tagtgaggag 2040
   gcttttttgg aggcctaggc ttttgcaaaa agctcccggg agcttgtata tccattttcg 2100
   gatctgatca agagacagga tgaggatcgt ttcgcatgat tgaacaagat ggattgcacg 2160
   caggttctcc ggccgcttgg gtggagaggc tattcggcta tgactgggca caacagacaa 2220
   tcggctgctc tgatgccgcc gtgttccggc tgtcagcgca ggggcgcccg gttctttttg 2280
   tcaagaccga cctgtccggt gccctgaatg aactgcagga cgaggcagcg cggctatcgt 2340
   ggctggccac gacgggcgtt ccttgcgcag ctgtgctcga cgttgtcact gaagcgggaa 2400
   gggactggct gctattgggc gaagtgccgg ggcaggatct cctgtcatct caccttgctc 2460
   ctgccgagaa agtatccatc atggctgatg caatgcggcg gctgcatacg cttgatccgg 2520
   ctacctgccc attcgaccac caagcgaaac atcgcatcga gcgagcacgt actcggatgg 2580
   aagccggtct tgtcgatcag gatgatctgg acgaagagca tcaggggctc gcgccagccg 2640
   aactgttcgc caggctcaag gcgcgcatgc ccgacggcga ggatctcgtc gtgacccatg 2700
   gcgatgcctg cttgccgaat atcatggtgg aaaatggccg cttttctgga ttcatcgact 2760
   gtggccggct gggtgtggcg gaccgctatc aggacatagc gttggctacc cgtgatattg 2820
   ctgaagagct tggcggcgaa tgggctgacc gcttcctcgt gctttacggt atcgccgctc 2880
   ccgattcgca gcgcatcgcc ttctatcgcc ttcttgacga gttcttctga gcgggactct 2940
   ggggttcgaa atgaccgacc aagcgacgcc caacctgcca tcacgagatt tcgattccac 3000
   cgccgccttc tatgaaaggt tgggcttcgg aatcgttttc cgggacgccg gctggatgat 3060
   cctccagcgc ggggatctca tgctggagtt cttcgcccac cccaacttgt ttattgcagc 3120
   ttataatggt tacaaataaa gcaatagcat cacaaatttc acaaataaag catttttttc 3180
   actgcattct agttgtggtt tgtccaaact catcaatgta tcttatcatg tctgtatacc 3240
   gtcgacctct agctagagct tggcgtaatc atggtcatag ctgtttcctg tgtgaaattg 3300
   ttatccgctc acaattccac acaacatacg agccggaagc ataaagtgta aagcctgggg 3360
   tgcctaatga gtgagctaac tcacattaat tgcgttgcgc tcactgcccg ctttccagtc 3420
   gggaaacctg tcgtgccagc tgcattaatg aatcggccaa cgcgcgggga gaggcggttt 3480
   gcgtattggg cgctcttccg cttcctcgct cactgactcg ctgcgctcgg tcgttcggct 3540
   gcggcgagcg gtatcagctc actcaaaggc ggtaatacgg ttatccacag aatcagggga 3600
   taacgcagga aagaacatgt gagcaaaagg ccagcaaaag gccaggaacc gtaaaaaggc 3660
   cgcgttgctg gcgtttttcc ataggctccg cccccctgac gagcatcaca aaaatcgacg 3720
   ctcaagtcag aggtggcgaa acccgacagg actataaaga taccaggcgt ttccccctgg 3780
   aagctccctc gtgcgctctc ctgttccgac cctgccgctt accggatacc tgtccgcctt 3840
   tctcccttcg ggaagcgtgg cgctttctca tagctcacgc tgtaggtatc tcagttcggt 3900
   gtaggtcgtt cgctccaagc tgggctgtgt gcacgaaccc cccgttcagc ccgaccgctg 3960
   cgccttatcc ggtaactatc gtcttgagtc caacccggta agacacgact tatcgccact 4020
   ggcagcagcc actggtaaca ggattagcag agcgaggtat gtaggcggtg ctacagagtt 4080
   cttgaagtgg tggcctaact acggctacac tagaagaaca gtatttggta tctgcgctct 4140
   gctgaagcca gttaccttcg gaaaaagagt tggtagctct tgatccggca aacaaaccac 4200
   cgctggtagc ggtttttttg tttgcaagca gcagattacg cgcagaaaaa aaggatctca 4260
   agaagatcct ttgatctttt ctacggggtc tgacgctcag tggaacgaaa actcacgtta 4320
   agggattttg gtcatgagat tatcaaaaag gatcttcacc tagatccttt taaattaaaa 4380
   atgaagtttt aaatcaatct aaagtatata tgagtaaact tggtctgaca gttaccaatg 4440
   cttaatcagt gaggcaccta tctcagcgat ctgtctattt cgttcatcca tagttgcctg 4500
   actccccgtc gtgtagataa ctacgatacg ggagggctta ccatctggcc ccagtgctgc 4560
   aatgataccg cgagacccac gctcaccggc tccagattta tcagcaataa accagccagc 4620
   cggaagggcc gagcgcagaa gtggtcctgc aactttatcc gcctccatcc agtctattaa 4680
   ttgttgccgg gaagctagag taagtagttc gccagttaat agtttgcgca acgttgttgc 4740
   cattgctaca ggcatcgtgg tgtcacgctc gtcgtttggt atggcttcat tcagctccgg 4800
   ttcccaacga tcaaggcgag ttacatgatc ccccatgttg tgcaaaaaag cggttagctc 4860
   cttcggtcct ccgatcgttg tcagaagtaa gttggccgca gtgttatcac tcatggttat 4920
   ggcagcactg cataattctc ttactgtcat gccatccgta agatgctttt ctgtgactgg 4980
   tgagtactca accaagtcat tctgagaata gtgtatgcgg cgaccgagtt gctcttgccc 5040
   ggcgtcaata cgggataata ccgcgccaca tagcagaact ttaaaagtgc tcatcattgg 5100
   aaaacgttct tcggggcgaa aactctcaag gatcttaccg ctgttgagat ccagttcgat 5160
   gtaacccact cgtgcaccca actgatcttc agcatctttt actttcacca gcgtttctgg 5220
   gtgagcaaaa acaggaaggc aaaatgccgc aaaaaaggga ataagggcga cacggaaatg 5280
   ttgaatactc atactcttcc tttttcaata ttattgaagc atttatcagg gttattgtct 5340
   catgagcgga tacatatttg aatgtattta gaaaaataaa caaatagggg ttccgcgcac 5400
   atttccccga aaagtgccac ctgacgtc 5428
<210> 15
   <211> 178
   <212> PRT
   <213> Homo sapiens
<400> 15
   Met Lys Pro Lys Met Lys Tyr Ser Thr Asn Lys Ile Ser Thr Ala Lys
   1 5 10 15
   Trp Lys Asn Thr Ala Ser Lys Ala Leu Cys Phe Lys Leu Gly Lys Ser
   20 25 30
   Gln Gln Lys Ala Lys Glu Val Cys Pro Met Tyr Phe Met Lys Leu Arg
   35 40 45
   Ser Gly Leu Met Ile Lys Lys Glu Ala Cys Tyr Phe Arg Arg Glu Thr
   50 55 60
   Thr Lys Arg Pro Ser Leu Lys Thr Gly Arg Lys His Lys Arg His Leu
   65 70 75 80
   Val Leu Ala Ala Cys Gln Gln Gln Ser Thr Val Glu Cys Phe Ala Phe
   85 90 95
   Gly Ile Ser Gly Val Gln Lys Tyr Thr Arg Ala Leu His Asp Ser Ser
   100 105 110
   Ile Thr Gly Ile Ser Pro Ile Thr Glu Tyr Leu Ala Ser Leu Ser Thr
   115 120 125
   Tyr Asn Asp Gln Ser Ile Thr Phe Ala Leu Glu Asp Glu Ser Tyr Glu
   130 135 140
   Ile Tyr Val Glu Asp Leu Lys Lys Asp Glu Lys Lys Asp Lys Val Leu
   145 150 155 160
   Leu Ser Tyr Tyr Glu Ser Gln His Pro Ser Asn Glu Ser Gly Asp Gly
   165 170 175
   Val Asp
<210> 16
   <211> 92
   <212> PRT
   <213> Homo sapiens
<400> 16
   Gly Lys Met Leu Met Val Thr Leu Ser Pro Thr Lys Asp Phe Trp Leu
   1               5                   10                  15
   His Ala Asn Asn Lys Glu His Ser Val Glu Leu His Lys Cys Glu Lys
               20                  25                  30
   Pro Leu Pro Asp Gln Ala Phe Phe Val Leu His Asn Met His Ser Asn
           35                  40                  45
   Cys Val Ser Phe Glu Cys Lys Thr Asp Pro Gly Val Phe Ile Gly Val
       50                  55                  60
   Lys Asp Asn His Leu Ala Leu Ile Lys Val Asp Ser Ser Glu Asn Leu
   65                  70                  75                  80
   Cys Thr Glu Asn Ile Leu Phe Lys Leu Ser Glu Thr
                   85                  90

## Description

### Introduction

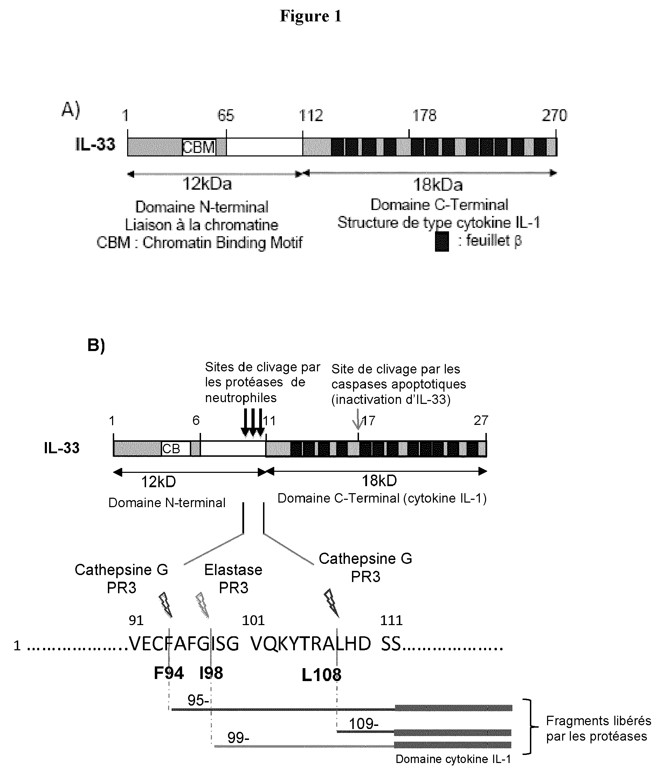

Les cytokines de la famille interleukine 1 (IL-1) jouent un rôle majeur dans un grand nombre de maladies auto-immunes, infectieuses et inflammatoires (Sims JE and Smith DE, Nature Rev Immunology 2010). L'interleukine 33 (IL-33), initialement nommée NF-HEV, est une nouvelle cytokine de la famille IL-1 comportant 270 acides aminés (Figure 1, NP_254274, SEQ ID NO :1). C'est un facteur nucléaire fortement exprimé dans les cellules endothéliales cuboïdales (Bakkevold E. et al., American Journal Pathology 2003). L'IL-33 présente, dans sa partie carboxy-terminale, des homologies structurales avec l'interleukine 1 (structure à 12 feuillets beta) (Lingel et al., Structure 2009) et se lie au récepteur orphelin ST2 (IL1-R4) (Chackerian AAet al., Journal of Immunology 2007). L'IL-33 est une protéine nucléaire associée à la chromatine (Carriere V. et al., PNAS 2007). Elle est exprimée de façon constitutive dans les cellules endothéliales des vaisseaux sanguins de la plupart des tissus, ainsi que dans des cellules épithéliales de tissus exposés à l'environnement comme la peau, l'estomac, les glandes salivaires ou les poumons (Moussion et al., PLoS ONE 2008).

Il est connu que l'IL-33 est libérée sous sa forme entière (IL-33ₐₐ₁₋₂₇₀), biologiquement active, en cas de dommage ou de nécrose cellulaires. L'IL-33 entière est en effet capable de se lier au récepteur ST2 (Cayrol and Girard, PNAS 2009) et d'activer les cellules cibles, notamment les cellules du système immunitaire : les lymphocytes Th2, les mastocytes, les cellules tueuses NK et iNKT, les basophiles et les éosinophiles (pour revue, voir Liew et al., Nature Rev Immunology 2010). L'activation du récepteur ST2 sur les cellules cibles induit, selon les cellules, la sécrétion de cytokines Th2 (IL-4, IL-5, IL-13) et/ou de cytokines pro-inflammatoires (TNF-α, IL-1, IL-6, IFN-γ). De plus, plusieurs études récentes ont montré la capacité d'IL-33 à recruter des cellules du système immunitaire, comme les neutrophiles (Alves-Filho et al., Nature Medicine 2010) sur les sites inflammatoires ou infectieux. Selon ces résultats, l'IL-33 pourrait donc servir de signal de danger pour alerter le système immunitaire après une infection ou un traumatisme des cellules endothéliales ou épithéliales (Moussion et al., PLoS ONE 2008; Cayrol and Girard, PNAS 2009).

L'IL-33 a un effet protecteur dans certaines pathologies, tandis qu'elle est un facteur d'aggravation dans d'autres. Par exemple, l'IL-33 a un rôle protecteur dans des maladies infectieuses comme le sepsis (Alves-Filho et al., Nature Medicine 2010), la kératite à Pseudomonas (Hazlett et al., Invest Ophtalmol Vis Sci. 2010) ou l'infection par des nématodes (Humphreys et al., Journal of Immunology 2008). Ce rôle protecteur est également démontré dans l'athérosclerose (Miller et al., J. Exp. Med 2008), l'infarcus du myocarde (Seki et al., Circ Heart Fail 2009) l'inflammation des tissus adipeux en cas d'obésité (Miller et al, Circ Res 2010) et le cancer (WO2005/079844). Ces données suggèrent qu'IL-33 pourrait être utilisée comme agent thérapeutique pour ces pathologies. A l'opposé, il a été démontré qu'IL-33 exacerbait les effets physiopathologiques dans d'autres types de maladies comme la polyarthrite rhumatoïde (Xu et al., Journal of Immunology 2010), l'inflammation des voies aériennes de type allergiques comme l'asthme (Stolarski et al., Journal of Immunology 2010), la fibrose cutanée (Rankin et al., J. immunol. 2010) ou le choc anaphylactique (Pushparaj et al., PNAS 2008), faisant d'IL-33 une cible thérapeutique potentielle pour ces pathologies.

Ainsi, ces études ont montré, à la fois chez l'homme et dans des modèles animaux, que l'administration *in vivo* d'IL-33 pourrait favoriser la guérison de nombreuses maladies, en particulier des maladies inflammatoires chroniques, des maladies de type allergiques, des maladies cardio-vasculaires, des maladies infectieuses, le cancer et la maladie d'Alzheimer (pour revue voir Liew et al., Nature Rev Immunology 2010).

Il est à noter que les auteurs des différentes études mentionnées ci-dessus ont utilisé, pour leurs études *in vivo,* une forme tronquée artificielle de l'IL-33 (IL-33ₐₐ₁₁₂₋₂₇₀), qui contient le domaine de type « cytokine IL-1 » de l'IL-33 (acides aminés 112 à 270), et qui est dépourvue du domaine N-terminal de la protéine entière (acides aminés 1 à 111) (cf. figure 1A). La communauté scientifique a longtemps cru que cette forme tronquée IL-33ₐₐ₁₁₂₋₂₇₀ pouvait être générée *in vivo.* En effet, il avait été suggéré qu'une forme IL-33ₐₐ₁₁₂₋₂₇₀, correspondant au domaine C-terminal de type « Cytokine IL-1 », pouvait être générée suite au clivage de l'IL-33 par la caspase 1 après le résidu Ser111 (Schmitz et al., Immunity 2005); il était alors acquis pour la communauté scientifique que la maturation d'IL-33 par la caspase 1 était possible, produisant la forme tronquée active (IL-33ₐₐ₁₁₂₋₂₇₀). Cependant, il a été récemment établi que le clivage par les caspases ne résulte pas en une activation mais en une inactivation de la protéine, car ces protéases clivent la protéine IL-33 dans le domaine de type cytokine IL-1, entre les 4ième et 5ième feuillets β, au niveau de la séquence DGVD (aa175-178). Le clivage de l'IL-33 par les caspases apoptotiques pourrait être un mécanisme sélectionné pour éliminer les activités pro-inflammatoires de l'IL-33 pendant l'apoptose, un processus qui n'entraîne pas d'inflammation *in vivo.*

Schmitz et al. (Immunity 2005) enseigne que l'IL-33 serait soit-disant clivé par la caspase 1 après le résidu Ser111 *in vivo,* produisant ainsi la forme clivée IL-33ₐₐ₁₁₂₋₂₇₀ et suggère que cette forme clivée a une action biologique sur le récepteur à l'IL-1 (ST2).

Résultat plus important encore, il a pu être démontré que la forme mature IL-33ₐₐ₁₁₂₋₂₇₀, utilisée dans de nombreuses études, n'était pas générée par l'action de la caspase 1 *in vitro* et donc, *a fortiori, in vivo.* Cette forme tronquée IL-33ₐₐ₁₁₂₋₂₇₀ n'existe donc pas *in vivo* (Cayrol and Girard, PNAS 2009, Lüthi et al., Immunity 2009 ; Talabot-Ayer et al., J. Biol. Chem. 2009 ; Ali et al., Biochem Biophys Res Commun 2010), et n'a donc jamais été rencontrée par le système immunitaire humain.

Plus précisément, Cayrol and Girard (PNAS 2009), et Lüthi et al. (Immunity 2009) (ainsi que la demande WO 2008/132709, laquelle se base sur les résultats expérimentaux publiés dans Lüthi et al.) décrivent que le clivage de l'IL-33 n'a pas lieu après la Ser111 mais après le résidu Asp178 (D2), et que ce clivage génère des fragments de l'IL-33 qui sont inactifs *in vitro* et *in vivo.* Ces documents suggèrent que ce clivage par les caspases 3 et 7 permet en fait de contrôler *in vivo* l'activité de l'IL-33 dans les cellules apoptotiques.

Son utilisation en thérapie humaine pourrait donc poser des problèmes importants, d'immunogénicité en particulier. L'utilisation d'une forme naturelle d'IL-33 est préférable pour les applications en thérapie humaine. Cependant, il n'a, à ce jour, pas été possible d'exprimer la protéine IL-33 entière dans des quantités compatibles avec la tenue d'essais *in vivo,* et encore moins avec la production industrielle d'un médicament biothérapeutique.

Il convenait donc d'identifier une forme active de l'IL-33 humaine, qui i) soit active, au moins autant, voire plus active que la forme entière, ii) soit générée de façon naturelle (et donc *a priori* peu ou pas immunogène), et iii) puisse être produite à grande échelle sous forme recombinante (par milligrammes voire par grammes), afin d'être utilisée comme traitement à moyen et long terme chez l'homme.

Or aucune protéase pouvant induire la maturation de l'IL-33 humaine, de façon naturelle, en une forme plus active que la forme entière n'avait été identifiée à ce jour.

Les présents inventeurs ont répondu à ce besoin, en démontrant que l'IL-33 entière est clivée de façon naturelle par trois protéases de neutrophiles, la cathepsine G, l'élastase et la protéinase 3 humaines. Ces protéases libérent des fragments carboxy-terminaux de tailles supérieures à la forme tronquée artificielle IL-33ₐₐ₁₁₂₋₂₇₀, lesdits fragments ayant une activité biologique supérieure, tant *in vitro* qu'*in vivo,* à celle la forme entière IL-33ₐₐ₁₋₂₇₀. A la différence de la forme IL-33ₐₐ₁₋₂₇₀, ces nouveaux fragments de l'IL-33 sont facilement exprimés dans les cellules bactériennes comme *E.Coli.* Ils ont l'avantage d'être obtenus de façon naturelle, et sont plus actifs *in vivo* que la forme tronquée artificielle de l'IL-33 utilisée jusqu'à présent (IL-33ₐₐ₁₁₂₋₂₇₀) (cf. exemple d) ci-dessous).

De par leurs propriétés, ces nouveaux fragments de l'IL-33 pourraient être produits dans les industries de biotechnologie afin d'être utilisés notamment en santé humaine (traitements de maladies humaines dans lesquelles l'IL-33 a un effet protecteur) ou pour la recherche scientifique.

### Légendes des figures

La figure 1 A décrit la structure primaire de la protéine IL-33 humaine entière. Les domaines fonctionnels (liaison à la chromatine, cytokine IL-1) sont indiqués. La figure 1 B présente les sites de clivage par les protéases de neutrophile cathepsine G (Cat G), Elastase 2 (Elastase) et Protéase 3 (PR3) en amont du domaine C-terminal de type « cytokine IL-1 » ainsi que les fragments libérés par l'action de ces protéases (fragments IL-33ₐₐ₉₅₋₂₇₀, IL-33ₐₐ₉₉₋₂₇₀ et IL-33ₐₐ₁₀₉₋₂₇₀).

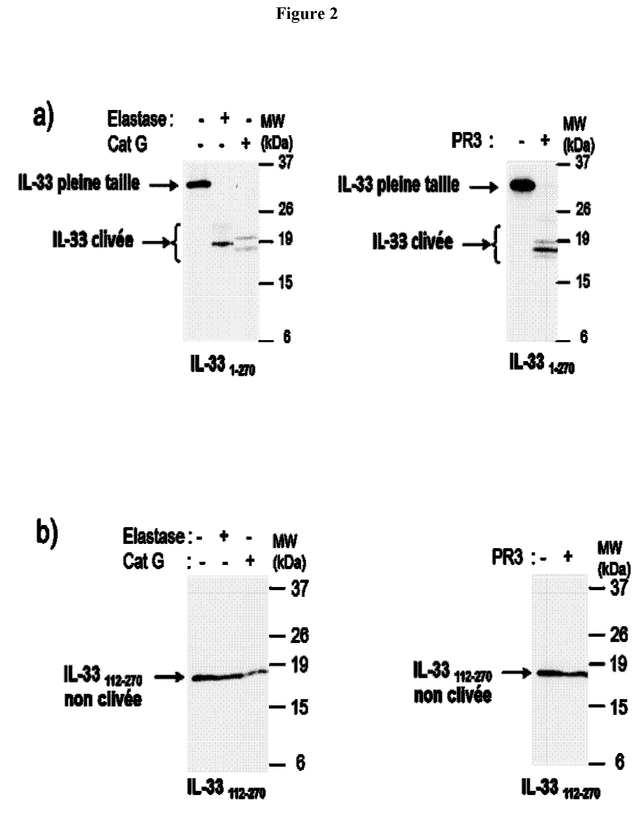

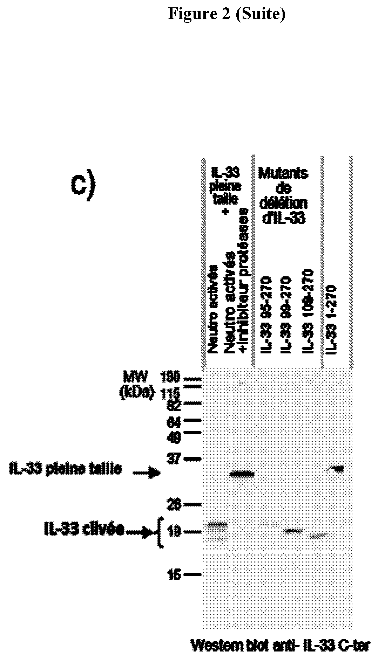

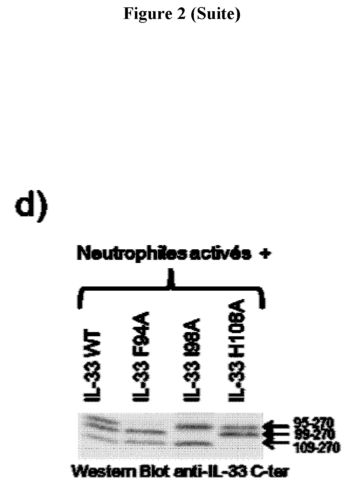

La figure 2 A présente les résultats des western-blots démontrant que l'interleukine IL-33 humaine entière est clivée *in vitro* par les protéases recombinantes Cathepsine G (Cat G), Elastase 2 (Elastase) et Protéase 3 (PR3), au contraire de la forme tronquée IL-33ₐₐ₁₁₂₋₂₇₀, qui reste intacte en présence de ces protéases (Figure 2B). La figure 2C présente les résultats démontrant que l'IL-33 humaine est le substrat de ces mêmes protéases humaines extraites directement de neutrophiles humains *ex vivo.* La figure 2D montre que les fragments IL-33ₐₐ₉₅₋₂₇₀, IL-33ₐₐ₉₉₋₂₇₀ et IL-33ₐₐ₁₀₉₋₂₇₀ ne sont pas libérés par les protéases de neutrophiles lorsque les sites de clivage correspondant sont mutés.

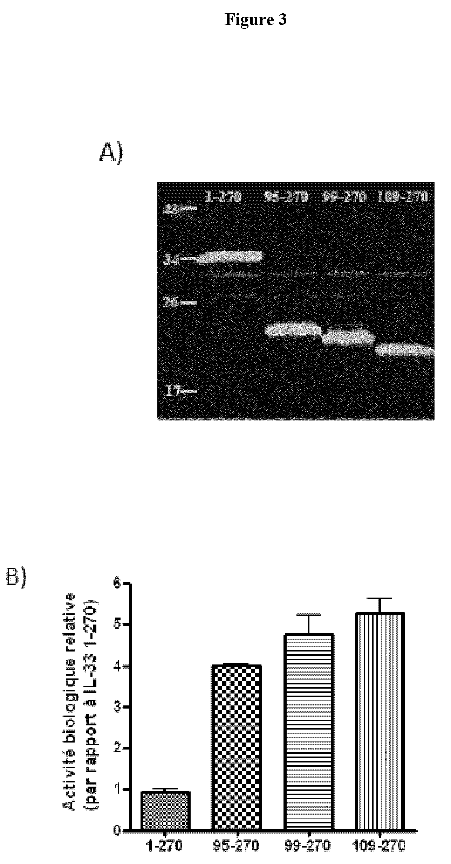

La figure 3 A présente l'analyse quantitative des protéines recombinantes IL-33ₐₐ₁₋₂₇₀, IL-33ₐₐ₉₅₋₂₇₀, IL-33ₐₐ₉₉₋₂₇₀ et IL-33ₐₐ₁₀₉₋₂₇₀ par western blot, tandis que la figure 3 B présente l'activité de ces différentes protéines *in vitro* sur la sécrétion d'IL-6 par des cellules mastocytaires MC/9.

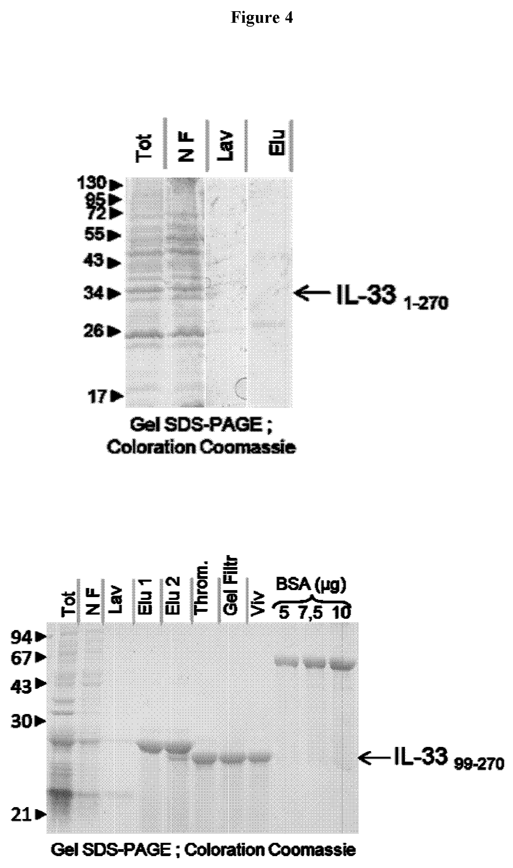

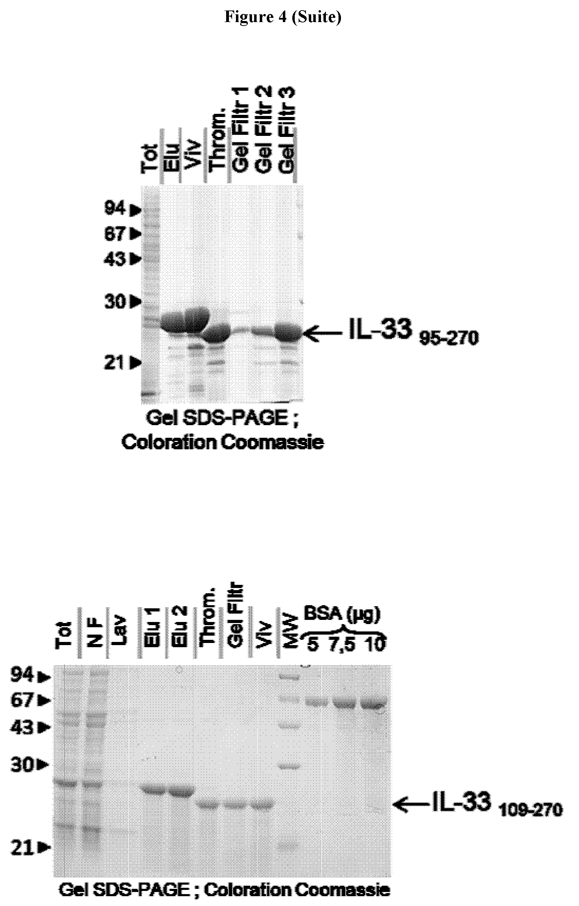

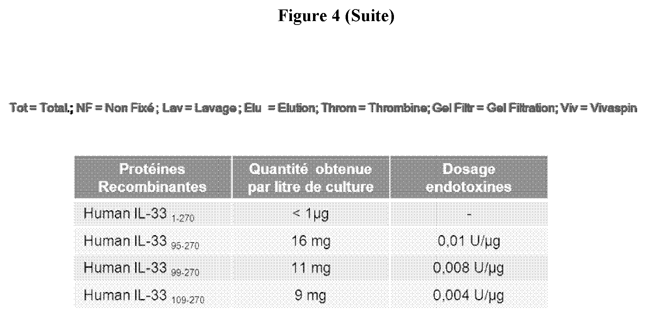

La figure 4 présente les profils d'expression des trois fragments polypeptidiques de l'invention, IL-33ₐₐ₉₅₋₂₇₀, IL-33ₐₐ₉₉₋₂₇₀ et IL-33ₐₐ₁₀₉₋₂₇₀ obtenus par voie recombinante dans des cellules bactériennes (le vecteur utilisé est le vecteur pET15b et la souche *d'E. coli* est la souche BL21, pour plus de détails voir l'exemple c) ci-dessous). Plusieurs milligrammes de protéine recombinante purifiée ont été obtenus à partir d'un litre de culture d'*E. coli* (cf. tableau).

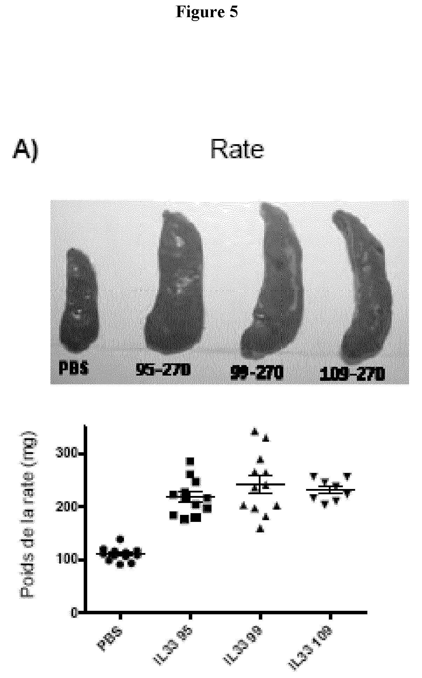

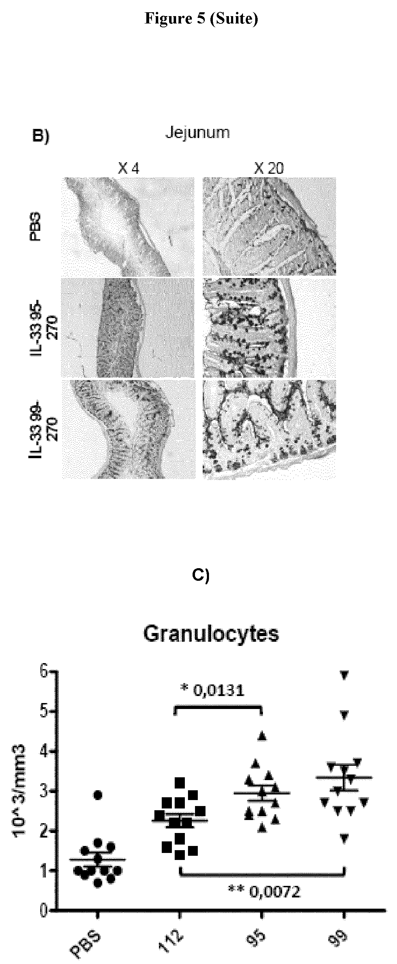

La figure 5 présente les résultats obtenus *in vivo* avec les fragments polypeptidiques de l'invention. Les rates de souris ayant reçu quotidiennement une injection de ces fragments pendant 7 jours ont été prélevées (A) et leur jejunum a été analysé par marquage PAS-alcian blue (B). Le nombre de granulocytes dans le sang a été mesuré au bout des 7 jours (C).

### Description de l'invention

Le but de la présente invention était d'identifier un fragment actif de l'IL-33 humaine (au moins autant, voire plus actif que la forme entière, et préférentiellement au moins aussi actif que IL-33ₐₐ₁₁₂₋₂₇₀), qui corresponde à une forme naturelle (et donc *a priori* peu ou pas immunogène) et qui puisse être produit à grande échelle sous forme recombinante (de l'ordre du milligramme voire du gramme) et donc être utilisable pour des applications en santé (traitements à moyen et long terme chez l'homme) et en biotechnologie (recherche scientifique).

Les neutrophiles sont les premiers leucocytes circulants à migrer sur le site de l'inflammation et constituent la première ligne de défense contre les agents pathogènes. Outre leur rôle de phagocytes, il peuvent assurer une défense innée en relarguant des protéines granulaires, en particulier les protéases Cathepsine G, Elastase 2 ou Protéinase 3, dans le milieu extra-cellulaire.

Les présents inventeurs ont pu démontrer, pour la première fois, que l'action de la cathepsine G ou de la protéinase 3 des leucocytes libère les fragments polypeptidiques IL-33ₐₐ₉₅₋₂₇₀ et IL-33ₐₐ₁₀₉₋₂₇₀ du domaine N-terminal de l'IL-33 humaine. L'action de l'élastase 2 des neutrophiles ou de la protéase 3 libère quant à elles le fragment polypeptidique IL-33ₐₐ₉₉₋₂₇₀ du domaine N-terminal de l'IL-33 humaine. Les sites de clivage de ces protéases ont pu être déterminés très précisément, mettant ainsi en évidence trois nouveaux fragments de l'IL-33, IL-33ₐₐ₉₅₋₂₇₀, IL-33ₐₐ₉₉₋₂₇₀ et IL-33ₐₐ₁₀₉₋₂₇₀, qui n'avaient jamais été identifiés à ce jour (cf. figures 1B et 2). A l'inverse du fragment IL-33ₐₐ₁₁₂₋₂₇₀ utilisé jusqu'à présent, ces fragments résultent du clivage naturel de l'IL-33 entière par les protéases des neutrophiles chez l'homme, et sont donc physiologiques.

De façon surprenante, ces fragments de l'IL-33 (IL-33ₐₐ₉₅₋₂₇₀, IL-33ₐₐ₉₉₋₂₇₀ et IL-33ₐₐ₁₀₉₋₂₇₀) présentent *in vitro* et *in vivo* une activité biologique supérieure à la forme entière de l'IL-33 humaine et/ou à la forme tronquée IL-33ₐₐ₁₁₂₋₂₇₀ utilisée jusqu'ici: en effet, ces trois nouveaux fragments d'IL-33 induisent *in vitro* la sécrétion de quatre fois plus d'IL-6 que la forme entière de l'IL-33 dans une lignée mastocytaire murine MC/9 (figure 3B) et une injection quotidienne de peptides à des souris pendant 7 jours entraîne une réaction inflammatoire se traduisant par un doublement du poids de la rate au bout de 7 jours, et une augmentation significative du nombre de granulocytes (majoritairement des neutrophiles) dans le sang des souris, par rapport à la forme tronquée IL-33ₐₐ₁₁₂₋₂₇₀ (cf. exemple d) ci-dessous).

Enfin, contrairement à la forme entière de l'IL-33 humaine, ces fragments polypeptidiques de l'IL-33 (IL-33ₐₐ₉₅₋₂₇₀, IL-33ₐₐ₉₉₋₂₇₀ et IL-33ₐₐ₁₀₉₋₂₇₀) sont aisément exprimés par les cellules classiquement utilisées pour la production de protéines recombinantes (cellules *E. coli* par exemple), et des taux de 9 mg par litre de culture cellulaire sont obtenus (voir exemple c) ci-dessous).

Dans un premier aspect, la présente invention concerne donc un fragment polypeptidique isolé de l'interleukine 33 humaine (hIL-33), biologiquement actif, choisi parmi les fragments ayant pour séquence les acides aminés 95 à 270 (SEQ ID NO :2), 99 à 270 (SEQ ID NO :3) ou 109 à 270 (SEQ ID NO :4) de l'interleukine 33 humaine (hIL-33), lesdits fragments étant des produits naturels de clivage de hIL-33 par au moins une protéase des neutrophiles humains.

Dans un mode de réalisation particulier de l'invention, ladite protéase des neutrophiles humains est choisie dans le groupe constitué par : la cathepsine G, la protéinase 3 des leucocytes, et l'élastase 2 des neutrophiles.

Plus précisément, la présente invention vise le fragment polypeptidique IL-33ₐₐ₉₅₋₂₇₀ ayant la séquence SEQ ID NO :2, qui est un produit naturel de clivage de hIL-33 par la Cathepsine G et la protéinase 3 des leucocytes, le fragment polypeptidique IL-33ₐₐ₉₉₋₂₇₀ ayant la séquence SEQ ID NO :3, qui est un produit naturel de clivage de hIL-33 par la protéinase 3 des leucocytes et l'élastase 2 des neutrophiles, et enfin le fragment polypeptidique IL-33ₐₐ₁₀₉₋₂₇₀ ayant la séquence SEQ ID NO :4, qui est un produit naturel de clivage de hIL-33 par la Cathepsine G et la protéinase 3 des leucocytes.

Ces trois fragments résultent donc du clivage de l'IL-33 humaine entière par des protéases naturellement présentes chez l'homme, et sont donc naturels. Ils peuvent être générés naturellement chez l'homme et ne sont donc pas *a priori* immunogènes. En d'autres termes, ces fragments peuvent être reconnus comme des antigènes du soi par le système immunitaire humain, et ne déclenchent donc pas, lorsqu'ils sont injectés chez des êtres humains, de réaction immunitaire visant à leur propre rejet. Ils sont donc tolérés par les individus dans lesquels ils sont injectés, à court, moyen et long terme (typiquement, pendant toute la durée de vie du patient). Il est rappelé ici que la non-immunogénicité du fragment polypeptidique de l'invention n'affaiblit en rien sa faculté à stimuler et à recruter les cellules du système immunitaire (lymphocytes Th2, mastocytes, NK et iNKT, basophiles et éosinophiles) de l'individu dans lequel il est injecté, à l'instar de l'interleukine IL-33 entière.

On entend par « cathepsine G » au sens de la présente invention une enzyme appartenant à la famille des protéases S1 (ou peptidases SI). Son activité enzymatique est de préférence caractérisée par le numéro EC 3.4.21.20. Elle est notamment capable de cliver des chimiokines comme CXCL5 (Nufer O. et al., Biochemistry 1999) ou des récepteurs membranaire comme PAR4 (Sambrano G.R. et al., J. Biol. Chem. 2000). Elle possède une triade catalytique (sérine, histidine et aspartate), caractéristique des protéases à sérine des mammifères. La compilation de différentes données a permis de définir des sites de clivage préférentiels (Burster T., et al Molecular Immunology 2010). De façon caractéristique, des résidus aromatiques (Phe, Tyr) ou chargés (Lys, Arg) sont souvent retrouvés en position P1 (position de l'acide aminé situé juste en amont de la liaison clivée) (Korkmaz, B. et al., Pharmacological Reviews 2010). Cette enzyme est retrouvée de préférence dans les granules azurophiles des leucocytes polymorphonucléaires neutrophiles. Elle participe à la destruction et à la digestion des pathogènes présents dans les tissus conjonctifs ou aux sites d'inflammation (Kargi H.A. et al, J. Histochem. Cytochem 1990). Chez l'homme, elle est codée par le gène CTSG. Elle a de préférence la séquence décrite sous le numéro d'accession HUMCAPG J04990.1. De manière encore plus préférée, elle a la séquence SEQ ID NO :6. Les présents inventeurs ont pu démontrer, pour la première fois, que l'action de la cathepsine G de séquence SEQ ID NO :6 libère les fragments polypeptidiques IL-33ₐₐ₉₅₋₂₇₀ et IL-33ₐₐ₁₀₉₋₂₇₀ du domaine N-terminal de l'IL-33 humaine.

On entend par « protéinase 3 des leucocytes » au sens de la présente invention la myéloblastine, la protéinase 4 des neutrophiles, la protéine 7 des granules azurophiles, ou encore l'auto-antigène de la granulomatose de Wegener. Elle sera appelée « protéinase 3 » (PRTN3) dans le cadre de la présente demande. Son activité enzymatique est de préférence caractérisée par le numéro EC 3.4.21.76. Cette enzyme, appartenant également à la famille des protéases S1, est capable d'hydrolyser des protéines, et notamment l'élastine (Rao N.V. et al, PNAS 1991). Les substrats préférentiels de la PR3 possèdent, en position P1, des acides aminés hydrophobes (Val, Cys, Ala, Met, Leu, Ser) (Korkmaz et al., Pharmacological Reviews 2010) ou polaires (Asn, Cys, Glu, Gln, Ser, Thr, Trp, Tyr) (Hajjar E et al., FEBS Letters 2007). De préférence, la protéinase 3 utilisée dans l'invention est l'enzyme humaine, par exemple celle référencée sous le numéro d'accession NCBINP_002768.3. De manière encore plus préférée, la protéinase 3 utilisée dans l'invention a la séquence SEQ ID NO :7. Les présents inventeurs ont pu démontrer que l'action de la protéase 3 de séquence SEQ ID NO :7 sur l'IL-33 humaine libère les fragments polypeptidiques IL-33ₐₐ₉₅₋₂₇₀, IL-33ₐₐ₁₀₉₋₂₇₀ et IL-33ₐₐ₉₉₋₂₇₀.

On entend par « élastase 2 des neutrophiles » au sens de la présente invention, la medullasine, ou encore l'élastase des leucocytes, est codée par le gène ELANE. Elle sera appelée « élastase 2 » dans le cadre de la présente demande. Son activité enzymatique est de préférence caractérisée par le numéro EC 3.4.21.37. Cette enzyme est capable de cliver l'élastine (Bieth J.G.., J. Soc. Biol. 2001). Concernant les sites de clivage spécifiques, on note une préférence de petits résidus hydophobes en position P1 (Val, Cys, Ala, Met, Leu, Ser) (Korkmaz et al ., 2010 ; Hajjar E. et al., 2007). De préférence, l'élastase 2 utilisée dans la présente invention est l'enzyme humaine, par exemple la protéine référencée sous le numéro d'accession NCBI NP_001963.1. De manière encore plus préférée, l'élastase 2 de l'invention a pour séquence SEQ ID NO :8. Les présents inventeurs ont pu démontrer, pour la première fois, que l'action de l'élastase 2 des neutrophiles sur l'IL-33 humaine libère le fragment polypeptidique IL-33ₐₐ₉₉₋₂₇₀.

Ces trois protéases (cathepsine G, protéinase 3 et élastase 2) font partie de la famille des « protéases à sérine de neutrophiles ». Dans un mode de réalisation particulier, la cathepsine G ci-dessus mentionnée a la séquence SEQ ID NO :6, la protéinase 3 a la séquence SEQ ID NO :7, et l'élastase 2 a la séquence SEQ ID NO :8. Les homologues de ces protéases peuvent également être visées. On entend ici par « homologues » les protéases ayant une séquence identique à au moins 80 %, de préférence à 85%, de préférence à 87 %, de préférence à 90 %, de préférence à 92 %, de préférence à 95 %, de préférence à 98 % et de manière encore plus préférée à 99% aux SEQ ID NO :6, SEQ ID NO :7 et SEQ ID NO :8 et conservant les activité enzymatiques des protéases élastase 2, protéinase 3 et cathepsine G correspondantes, telles que définies ci-dessus.

Au sens de la présente invention, le terme « interleukine 33 humaine » (IL-33) désigne une cytokine de la famille IL-1. Trois isoformes de l'IL-33 ont été identifiées chez l'homme : il s'agit de l'isoforme 1 (NP_254274), de l'isoforme 2 (NP_001186569), et de l'isoforme 3 (NP_001186570). L'IL-33 définie dans l'invention est l'isoforme 1 de l'IL-33, comportant 270 acides aminés, et identifiée sous le numéro d'accession NP_254274. Dans un mode de réalisation plus particulier, l'IL-33 de l'invention a la séquence SEQ ID NO :1.

Le terme « fragment polypeptidique » désigne dans la présente invention un enchaînement d'acides aminés reliés par des liaisons peptidiques, comportant au moins 50 acides aminés, de préférence au moins 100 acides aminés et au plus 250 acides aminés, de préférence entre environ 150 acides aminés et 180 acides aminés, de manière encore plus préférée entre environ 160 acides aminés et 175 acides aminés.

Lorsqu'il est utilisé par la suite, le terme « les fragments polypeptidiques » désigne chacun des trois fragments polypeptidiques IL-33ₐₐ₉₅₋₂₇₀, IL-33ₐₐ₉₉₋₂₇₀ et IL-33ₐₐ₁₀₉₋₂₇₀ tel que définis ci-avant.

Les fragments polypeptidiques de l'invention sont biologiquement actifs, c'est-à-dire qu'ils possèdent une activité biologique *in vitro* et/ou *in vivo,* par exemple sur l'activation et/ou le recrutement des cellules du système immunitaire.

De préférence, les fragments polypeptidiques de l'invention présentent au moins une (voire plusieurs) activité(s) biologique(s) améliorée(s) par rapport à la forme entière IL-33 et/ou par rapport à la forme tronquée de l'IL-33 déjà connue ayant la séquence SEQ ID NO :5 (IL-33ₐₐ₁₁₂₋₂₇₀). Cette activité biologique améliorée peut être par exemple l'activation et/ou le recrutement des cellules du système immunitaire. Ainsi, dans un mode de réalisation particulier, chacun des fragments polypeptidiques de l'invention a une activité de stimulation des cellules du système immunitaire augmentée par rapport à la forme entière de l'IL-33 humaine. Selon un autre mode de réalisation particulier, chacun des fragments polypeptidiques de l'invention a une activité de recrutement des cellules du système immunitaire augmentée par rapport à la forme tronquée de l'IL-33 humaine ayant la séquence SEQ ID NO :5 (IL-33ₐₐ₁₁₂₋₂₇₀). De préférence, lesdites cellules du système immunitaire sont les lymphocytes Th2, les mastocytes, les mastocytes, les cellules tueuses NK et iNKT, les basophiles et les éosinophiles.

Les propriétés de stimulation des cellules du système immunitaire peuvent être notamment évaluées *in vitro*, par exemple en mettant l'un desdits fragments au contact de cellules du système immunitaire et en mesurant leur état de stimulation. Dans ce test, les cellules du système immunitaire sont de préférence des basophiles ou des cellules mastocytaires, dont l'état de stimulation peut être estimé par exemple en mesurant la quantité de cytokines pro-inflammatoires, telles que l'IL-18, l'IL-4, l'IL-5, l'IL-6, l'IL-13 ou le GM-CSF, qu'ils sécrétent (Reen D.J., Methods Mol Biol. 1994). La mesure de la quantité de cytokines sécrétées peut se faire par de nombreuses techniques bien connues de l'homme du métier, et notamment par la technique ELISA. Un exemple d'un tel test est présenté ci-dessous (cf. exemple b)).

Dans un mode de réalisation préféré, les fragments polypeptidiques de l'invention présentent une meilleure activité *in vitro* de stimulation des cellules du système immunitaire par rapport à l'interleukine 33 humaine (hIL-33) native, et sont capables de stimuler la sécrétion d'au moins une de ces cytokines avec un ratio (par exemple défini comme la quantité de cytokine sécrétée par desdites cellules en présence du fragment de l'invention / quantité de cytokine sécrétée par les mêmes cellules, en présence de l'IL-33 sous forme entière) de préférence d'au moins deux, de manière encore plus préférée d'au moins trois, et de manière préférée entre toutes d'au moins quatre, par rapport à la forme entière de l'IL-33 humaine de SEQ ID NO :1.

Il est également possible d'évaluer *in vitro* la liaison de ces fragments polypeptidiques au récepteur ST2, afin de vérifier que ce dernier est bien stimulé par ces fragments.

Par ailleurs, les propriétés de recrutement de cellules du système immunitaire peuvent être évaluées *in vivo,* en injectant un des fragments peptidiques d'IL-33 dans la cavité péritonéale pendant 6 à 7 jours et en analysant la cellularité (éosinophiles, neutrophiles, granulocytes) dans la rate ou le sérum. Des changements pathologiques peuvent également être observés au niveau des poumons (présence de mucus), du tractus digestif (hyperplasie de cellules épithéliales et cellules caliciformes) ou du plasma (concentration accrue de cytokines comme IL-4, IL-5) (Schmitz J. et al., Immunity 2005 ; Lüthi A. et al., Immunity 2009). Un tel exemple est décrit ci-dessous (cf. exemple d)).

Dans un mode de réalisation préféré, les fragments de l'invention présentent une meilleure activité *in vivo* de recrutement des cellules du système immunitaire par rapport à la forme tronquée de l'IL-33 ayant pour séquence les acides aminés 112 à 270 (SEQ ID NO :5). Plus précisément, les fragments de l'invention sont de préférence capables d'induire une augmentation du poids de la rate et/ou d' augmenter la cellularité en granulocytes dans le sang des souris d'un facteur d'au moins 1,8, de manière encore plus préférée de 2, par rapport à l'injection d'un véhicule contrôle (PBS), et d'un facteur d'au moins 1,2, de manière encore plus préférée d'au moins 1,3, et de manière préférée entre toutes, d'au moins 1,4, par rapport à l'injection de la forme tronquée de l'IL-33 de séquence SEQ ID NO :5 (IL-33ₐₐ₁₁₂₋₂₇₀).

Au sens de la présente invention, un processus biologique a lieu « *in vitro »* lorsqu'il survient en dehors d'un organisme animal ou humain, par exemple dans du matériel de laboratoire (tube, éprouvette, eppendorfs, etc.), donc de façon artificielle. De préférence, ce processus ne fait pas intervenir de cellule animale ou humaine. A l'inverse, au sens de la présente invention, un processus biologique a lieu *« in vivo »* lorsqu'il survient dans un organisme humain ou animal. Au sens de la présente invention, un processus biologique est qualifié d' « *ex vivo »* lorsqu'il implique des cellules humaines ou animales vivantes qui sont cultivées en dehors de l'organisme dont elles sont issues (lignées cellulaires ou cellules primaires cultivées dans des matériels de laboratoire).

La présente demande décrit également les homologues des fragments ayant pour séquence les acides aminés 95 à 270 (SEQ ID NO :2), 99 à 270 (SEQ ID NO :3) ou 109 à 270 (SEQ ID NO :4) de l'interleukine 33 humaine (hIL-33).

Les « homologues » ci-dessus décrits sont des fragments polypeptidiques qui sont « homologues » à ceux ayant pour séquence SEQ ID NO :2, SEQ ID NO :3 et SEQ ID NO :4. Le terme « homologue » désigne ici un polypeptide ayant une séquence identique à au moins 80 %, de préférence à 85%, de préférence à 87 %, de préférence à 90 %, de préférence à 92 %, de préférence à 95 %, de préférence à 97 %, de préférence à 98 % et de manière encore plus préférée à 99% aux SEQ ID NO :2, SEQ ID NO :3 et SEQ ID NO :4 et ayant essentiellement la (ou les) même(s) activité(s) biologique(s) que celle(s) des fragments polypeptidiques correspondants.

De préférence, les « homologues » décrits dans la présente demande ne sont pas les fragments de l'IL33 humaine déjà connus, i.e. IL-33₁₋₁₇₈ et IL-33₁₇₉₋₂₇₀ de séquence SEQ ID NO:15 et SEQ ID NO :16 respectivement, ces fragments n'ayant pas d'activité *in vitro* et *in vivo* (cf. WO 2008/132709, Cayrol and Girard, PNAS 2009, Lüthi et al., Immunity 2009). Bien entendu, les « homologues » ne peuvent par ailleurs pas être la forme entière de l'IL33, ayant la SEQ ID NO : 1, ni la forme artificielle de l'IL-33, IL-33₁₁₂₋₂₇₀, ayant la SEQ ID NO : 5.

Par « ayant essentiellement la (ou les) même(s) activité(s) biologique(s)» ou « conserve essentiellement la (ou les) même(s) activité(s) biologique(s) », il est entendu ici que au moins 90 %, voire au moins 95 % d'au moins une des activités biologiques du fragment polypeptidique de SEQ ID NO : 2, SEQ ID NO :3 ou SEQ ID NO :4 est conservée pour le fragment ayant une séquence identique à au moins 80 %, de préférence à 85%, et de manière encore plus préférée à 90%. Avantageusement, cette activité biologique est l'activation des cellules du système immunitaire et peut être mesurée *in vitro* par exemple comme décrit ci-dessus. Alternativement, cette activité biologique est le recrutement des cellules du système immunitaire et peut être mesurée *in vivo* par exemple comme décrit ci-dessus. De manière encore plus préférée, les fragments polypeptidiques ayant une séquence identique à au moins 80 %, de préférence à 85%, et de manière encore plus préférée à 90% aux SEQ ID NO :2, SEQ ID NO :3 et SEQ ID NO :4 ont les mêmes capacités d'activation et de recrutement des cellules du système immunitaire que celles des fragments polypeptidiques correspondants.

Par « pourcentage d'identité » entre deux séquences d'acide aminé au sens de la présente invention, on entend désigner un pourcentage de résidus d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Les comparaisons de séquences entre deux séquences d'acide aminé sont traditionnellement réalisées en comparant ces séquences après les avoir alignées de manière optimale, ladite comparaison pouvant être réalisée par segment ou par « fenêtre de comparaison ». L'alignement optimal des séquences pour la comparaison peut être réalisé, outre manuellement, au moyen de certains algorithmes d'homologie locale (algorithme de Smith et Waterman, de Neddleman et Wunsch ou de Pearson et Lipman), au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide aminé est déterminé en comparant ces deux séquences alignées de manière optimale dans laquelle la séquence d'acide aminé à comparer peut comprendre des additions ou des délétions par rapport à la séquence de référence pour un alignement optimal entre ces deux séquences. Le pourcentage d'identité est calculé en déterminant le nombre de positions identiques pour lesquelles le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 sequences » (Tatusova et al., « Blast 2 sequences - a new tool for comparing protein and nucleotide sequences », FEMS Microbiol., 1999) disponible sur le site http://www.ncbi.nlm.nih.gov/gorf/bl2.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

Par « séquence d'acides aminés présentant au moins 80 %, de préférence 85%, de préférence 87 %, de préférence 90 %, de préférence 92 %, de préférence 95 %, de préférence 97 %, de préférence 98 % et de manière encore plus préférée 99% d'identité avec une séquence d'acides aminés de référence », on préfère celles présentant, par rapport à la séquence de référence, certaines modifications, en particulier une délétion, addition ou substitution d'au moins un acide aminé, une troncation ou un allongement, et qui conservent essentiellement la (ou les) même(s) activité(s) biologique(s) que celle(s) des polypeptides correspondants. Avantageusement, les activités d'activation et/ou de recrutement des cellules du système immunitaire sont essentiellement conservées.

Dans le cas d'une substitution, d'un ou plusieurs acide(s) aminé(s) consécutif(s) ou non consécutif(s), on préfère les substitutions dans lesquelles les acides aminés substitués sont remplacés par des acides aminés équivalents. L'expression « acides aminés équivalents » vise ici à désigner tout acide aminé susceptible d'être substitué à l'un des acides aminés de la structure de base sans cependant modifier essentiellement la ou les activités biologiques des fragments polypeptidiques correspondants. Avantageusement, l'activité d'activation et/ou le recrutement des cellules du système immunitaire sont conservées. Ces acides aminés équivalents peuvent être déterminés soit en s'appuyant sur leur homologie de structure avec les acides aminés auxquels ils se substituent, soit sur des résultats d'essais comparatifs d'activité biologique susceptibles d'être effectués entre les différents fragments. Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucine, l'isoleucine, la proline, la phénylalanine, la méthionine, le tryptophane, et la cystéine) (Hausman RE et al (2004). D.C: ASM Press). L'homme du métier déterminera facilement les régions du fragment peptidique qui peuvent tolérer un changement par référence aux plots Hopp/Woods et Kyte-Doolite, biens connus dans la technique.

La présente demande décrit en outre des fragments polypeptidiques modifiés chimiquement ou enzymatiquement pour améliorer leur stabilité, leur biodisponibilité et/ou leur activité. Avantageusement, les activités d'activation et/ou de recrutement des cellules du système immunitaire sont améliorées. L'homme du métier connait de nombreuses techniques pour modifier chimiquement ou enzymatiquement des acides aminés (voir également David L. Nelson et Michael M. Cox, Lehninger Principles of Biochemistry, 3rd edition, 2000).

De manière non limitative, on peut par exemple modifier un ou plusieurs acides aminés lysine (K) par :
- amidation : cette modification est simple à accomplir, la charge positive de la lysine étant substituée par des groupements hydrophobes (par exemple, acétyle ou phénylacétyle) ;
- amination : par formation d'amides secondaires à partir de l'amine primaire R = (CH2) 4-NH3+, par exemple en formant des groupements N- méthyle, N-allyle ou N-benzyle ;
- ou encore par formation des groupements N-oxyde, N-nitroso, N- dialkyl phosphoryle, N-sulfényle, ou N-glycoside.

On peut par ailleurs aussi ou alternativement modifier un ou plusieurs acides aminés thréonine (T) et/ou sérine (S) des peptides, notamment en introduisant au niveau du groupe OH de la chaîne latérale de la thréonine et/ou de la sérine, un groupement ester ou éther. L'estérification, opération simple, peut être réalisée à l'aide d'un acide carboxylique, d'un anhydride, par pontages, etc, pour former par exemple des acétates ou des benzoates. L'éthérification, qui donne des composés plus stables, peut être effectuée à l'aide d'un alcool, d'un halogénure, etc, pour former par exemple un méthyl éther ou un O-glycoside. On peut par ailleurs aussi ou alternativement modifier un ou plusieurs acides aminés glutamine (Q) par exemple par amination, en formant des amines secondaires ou tertiaires, notamment avec des groupements de type méthyl, éthyl, fonctionnalisés ou non. On peut par ailleurs aussi ou alternativement modifier un ou plusieurs acides aminés glutamate (E) et/ou aspartate (D), par exemple :
- par estérification, pour former des méthyl esters substitués ou non, des éthyl esters, benzyl esters, des thiols (esters activés), etc,
- par amidation, notamment pour former des groupements N, N diméthyle, nitroanilides, pyrrolidinyles, etc.

En revanche, il est préférable de ne pas modifier les acides aminés proline, qui participent à la structure secondaire des peptides, sachant en outre que les acides aminés G, A et M n'offrent généralement pas de possibilités de modification qui seraient manifestement intéressantes.

Les fragments de l'IL-33 identifiés par les présents inventeurs (IL-33ₐₐ₉₅₋₂₇₀, IL-33ₐₐ₉₉₋₂₇₀ et IL-33ₐₐ₁₀₉₋₂₇₀, et/ou leurs homologues), correspondant aux formes naturelles générées par les protéases des neutrophiles humains, peuvent être obtenues par génie génétique, en utilisant un système de production de protéine recombinante procaryote ou eucaryote, notamment en i) cultivant un microorganisme ou des cellules eucaryotes transformé(es) à l'aide d'une séquence nucléotidique codant ces différents fragments de l'IL-33 et ii) isolant les fragments de l'IL-33 produits par ledit microorganisme ou lesdites cellules eucaryotes. Cette technique est bien connue de l'homme du métier. Pour plus de détails la concernant, on pourra se référer à l'ouvrage ci-après : Recombinant DNA Technology I, Editors Ales Prokop, Raskesh K Bajpai ; Annals of the New-York Academy of Sciences, Volume 646, 1991. Les fragments peptidiques d'IL-33 de l'invention sont de préférence purifiés / isolés à partir de lysats de cellules et/ou de surnageants de cellules par lesquelles elles sont exprimées et/ou sécrétées. Cette purification peut se faire par tout moyen connu de l'homme du métier. De nombreuses techniques de purification sont décrites dans Voet D et Voet JG, Techniques de purification des protéines et des acides nucléiques, chapitre 6, Biochimie, 2nde édition.

Les systèmes de production de protéine recombinante utilisent des vecteurs nucléotidiques comprenant des acides nucléiques codant les polypeptides à synthétiser, qui sont introduits dans des cellules hôtes qui produisent lesdits polypeptides (pour plus de détails, se référer à « Recombinant DNA Technology I », Editors Ales Prokop, Raskesh K Bajpai ; Annals of the New-York Academy of Sciences, Volume 646, 1991).

Dans un deuxième aspect, l'invention vise donc un acide nucléique isolé codant au moins l'un des fragments polypeptidiques selon l'invention, c'est-à-dire codant au moins un polypeptide ayant une séquence choisie dans le groupe constitué en SEQ ID NO :2, SEQ ID NO :3 ou SEQ ID NO :4. La présente demande décrit en outre les acides nucléiques isolés codant les homologues desdits fragments polypeptidiques. Le terme « homologues » désigne ici encore un polypeptide ayant une séquence identique à au moins 80 %, de préférence à 85%, de préférence à 87 %, de préférence à 90 %, de préférence à 92 %, de préférence à 95 %, de préférence à 97 %, de préférence à 98 % et de manière encore plus préférée à 99% à l'une des séquences SEQ ID NO :2, SEQ ID NO :3 ou SEQ ID NO :4.

Dans un mode de réalisation préféré, un acide nucléique selon l'invention a entre 486 et 528 paires de bases. Plus préférentiellement, un acide nucléique de l'invention comprend une séquence choisie dans le groupe consistant en SEQ ID NO :9, SEQ ID NO :10 ou SEQ ID NO :11.

Dans un troisième aspect, l'invention vise également un vecteur comprenant au moins un acide nucléique selon l'invention. Ce vecteur peut servir à la production des fragments polypeptidiques de l'invention (vecteurs de clonage et/ou d'expression qui contiennent l'acide nucléique selon l'invention) ou encore pour transporter ledit fragment *in vivo* à des fins de thérapie génique.

De nombreux vecteurs dans lesquels on peut insérer une molécule d'acide nucléique d'intérêt afin de l'introduire et de la maintenir dans une cellule hôte eucaryote ou procaryote, sont connus; le choix d'un vecteur approprié dépend de l'utilisation envisagée pour ce vecteur (par exemple réplication de la séquence d'intérêt, expression de cette séquence, maintien de cette séquence sous forme extrachromosomique ou bien intégration dans le matériel chromosomique de l'hôte), ainsi que de la nature de la cellule hôte (par exemple, les plasmides sont de préférence introduits dans des cellules bactériennes, tandis que les YACs sont de préférence utilisés dans des levures). Ces vecteurs d'expression peuvent être des plasmides, des YACs, des cosmides, des rétrovirus, des épisomes dérivés d'EBV, et tous les vecteurs que l'homme du métier peut juger appropriés à l'expression desdites chaînes. Dans un mode de réalisation préféré de l'invention, le vecteur utilisé pour coder le fragment polypeptidique de l'invention est le vecteur pcDNA3.1 (+) de SEQ ID NO :14 (Invitrogen) pour les études *in vitro* et les vecteurs pET (Novagen) pour les productions à grande échelle chez la bactérie.

Les vecteurs selon l'invention comprennent l'acide nucléique codant au moins l'un des fragments polypeptidiques de l'invention, ou une séquence similaire, ainsi que les moyens nécessaires à son expression. On entend par « moyen nécessaire à l'expression d'un peptide », le terme peptide étant utilisé pour toute molécule peptidique, telle que protéine, polyprotéine, polypeptide, etc., tout moyen qui permet d'obtenir le peptide, tel que notamment un promoteur, un terminateur de transcription, une origine de réplication et de préférence un marqueur de sélection. Les moyens nécessaires à l'expression d'un peptide sont liés de façon opérationnelle à la séquence d'acide nucléique codant pour le fragment polypeptidique de l'invention. Par « liés de façon opérationnelle », on entend une juxtaposition desdits éléments nécessaires à l'expression et du gène codant pour le fragment polypeptidique de l'invention, lesquels sont en une relation telle que cela leur permet de fonctionner de façon attendue. Par exemple, il peut exister des bases supplémentaires entre le promoteur et le gène codant le fragment polypeptidique de l'invention tant que leur relation fonctionnelle est préservée. Les moyens nécessaires à l'expression d'un peptide peuvent être des moyens homologues, c'est-à-dire naturellement compris dans le génome du vecteur utilisé, ou bien être hétérologues, c'est-à-dire rajouté de façon artificielle à partir d'un autre vecteur et/ou organisme. Dans ce dernier cas, lesdits moyens sont clonés avec le fragment polypeptidique à exprimer. Des exemples de promoteurs hétérologues comprennent (i) les promoteurs viraux tels que le promoteur SV40 (Virus simien 40), le promoteur du gène de la thymidine-kinase du virus simplex de l'Herpès (TK-HSV-1), le LTR du virus du sarcome de Rous (RSV), le promoteur premier immédiat du cytomégolovirus (CMV) et le promoteur dernier majeur adénoviral (MLP), ainsi que (ii) tout promoteur cellulaire qui contrôle la transcription des gènes codant pour des peptides chez des eucaryotes supérieurs, tel que le promoteur du gène de phosphoglycérate-kinase (PGK) constitutif (Adra et al., Gene 1987), le promoteur des gènes spécifiques du foie alphal-antitrypsine et FIX et le promoteur SM22 spécifique des cellules du muscle lisse (Moessler et al., Development 1996). Les méthodes de suppression et d'insertion de séquences d'ADN dans des vecteurs d'expression sont largement connues de l'homme du métier et consistent notamment en des étapes de digestion enzymatique et ligature. Les vecteurs de l'invention peuvent également comprendre des séquences nécessaires au ciblage des peptides vers des compartiments cellulaires particuliers. Un exemple de ciblage peut être le ciblage vers le réticulum endoplasmique obtenu en utilisant des séquences d'adressage du type de la séquence leader issue de la protéine E3 de l'adénovirus (Ciernik I. F., et al., The Journal of Immunology, 1999).

Le terme « terminateur de transcription » désigne ici une séquence du génome qui marque la fin de la transcription d'un gène ou d'un opéron, en ARN messager. Le mécanisme de terminaison de la transcription est différent chez les procaryotes et chez les eucaryotes. L'homme du métier connait les signaux à utiliser en fonction des différents types cellulaires. Par exemple, si la cellule dans laquelle le vecteur va être introduit est une bactérie, il utilisera un terminateur Rho-indépendant (séquence répétée inversée suivie d'une série de T (uraciles sur l'ARN transcrit) ou un terminateur Rho-dépendant (constitués d'une séquence consensus reconnue par la protéine Rho).

Le terme « origine de réplication » (aussi appelée ori) est une séquence unique d'ADN permettant l'initiation de la réplication. C'est à partir de cette séquence que débute une réplication unidirectionnelle ou bidirectionnelle. L'homme du métier sait que la structure de l'origine de réplication varie d'une espèce à l'autre ; elle est donc spécifique bien qu'elles aient toutes certaines caractéristiques. Un complexe protéique se forme au niveau de cette séquence et permet l'ouverture de l'ADN et le démarrage de la réplication.

Les marqueurs de sélection sont bien connus de l'homme du métier. De manière préférée, il s'agit d'un gène codant une protéine conférant une résistance à un antibiotique.

Les vecteurs de l'invention, comprenant le ou les acide(s) nucléique(s) d'intérêt de l'invention, sont préparés par des méthodes couramment utilisées par l'homme du métier. Les clones résultant peuvent être introduits dans un hôte approprié par des méthodes standards connues de l'homme du métier pour introduire des polynucléotides dans une cellule hôte. De telles méthodes peuvent être la transformation à l'aide de dextrane, la précipitation par du phosphate de calcium, la transfection à l'aide de polybrène, la fusion de protoplastes, l'électroporation, l'encapsulation des polynucléotides dans liposomes, l'injection biolistique et la micro-injection directe d'DNA dans le noyau. On peut également associer ladite séquence (isolée ou insérée dans un vecteur plasmidique) avec une substance lui permettant de franchir la membrane des cellules hôtes, telle qu'un transporteur comme un nanotransporteur ou une préparation de liposomes, ou de polymères cationiques. En outre, on peut avantageusement combiner ces méthodes, par exemple en utilisant l'électroporation associée à des liposomes.

Dans un quatrième aspect, la présente invention concerne un microorganisme ou une cellule hôte eucaryote transformée de manière transitoire ou stable par - ou comprenant - l'un au moins des acides nucléiques de l'invention ou par l'un des vecteur de l'invention.

A titre d'exemples de microorganisme qui conviennent aux fins de l'invention, on peut citer les levures (Buckholz RG, Current Opinion in Biotechnology 1993) et les bactéries (Olins et Lee, Current Opinion in Biotechnology 1993). A titre d'exemples de cellules eucaryotes, on peut citer les cellules provenant d'animaux tels que les mammifères (Edwards CP et Aruffo A, Current Opinion in Biotechnology 1993), les reptiles, et équivalent. On peut également utiliser les cellules de plantes. Parmi les cellules de mammifère, on peut notamment utiliser les cellules d'ovaire de hamster chinois (CHO), du singe (cellules COS et Vero), du rein de hamster nain (cellules BHK), du rein de cochon (cellules PK 15) et du rein de lapin (cellules RK13, les lignées cellulaires humaines de l'ostéosacorme (cellules 143 B), les lignées cellulaires humaines HeLa et les lignées cellulaires humaines de l'hépatome (du type cellules Hep G2). Il est également possible d'utiliser des cellules d'insectes dans lesquelles on peut utiliser des procédés mettant en oeuvre des baculovirus par exemple (Luckow VA, Journal of Virology 1993). Dans un mode de réalisation préféré de l'invention, la cellule hôte utilisée pour produire le fragment de l'invention est une bactérie, de préférence la bactérie BL21.

L'homme du métier connait les conditions dans lesquelles cultiver ces cellules, ainsi que les conditions expérimentales nécessaires à l'expression des fragments polypeptidiques par ces cellules.

Sous un autre aspect, l'invention a également pour objet un procédé de production d'un fragment polypeptidique recombinant selon l'invention, caractérisé en ce qu'il comprend les étapes suivantes :
a) la culture dans un milieu et conditions de culture appropriés d'une cellule hôte selon l'invention ; et
b) l'isolement du fragment polypeptidique selon l'invention produit à l'étape a).

Le fragment peut être isolé (purifié) à partir de la cellule exprimant ledit fragment. Dans ce cas, une étape préalable de lyse desdites cellules pourra être nécessaire.

Les milieux et conditions de culture associées à chaque type cellulaire utilisé pour la production de protéines recombinantes sont bien connus de l'homme du métier.

L'isolement (ou la purification) du fragment polypeptidique de l'invention peut se faire par tout moyen connu de l'homme du métier. On citera par exemple la précipitation différentielle ou l'ultracentrifugation. Il peut être également avantageux de purifier les fragments d'intérêt par chromatographie d'échange ionique, par chromatographie d'affinité, par tamisage moléculaire, ou par isofocalisation. Toutes ces techniques sont décrites dans Voet D et Voet JG, Techniques de purification des protéines et des acides nucléiques, chapitre 6, Biochimie, 2nde édition.

Plus précisément, dans une première étape, le matériel d'où on veut extraire la protéine (tissu animal, partie de plantes, bactéries, etc) est généralement broyé. Divers appareils ("Waring Blender", appareil Potter-Eveljhem, "Polytron", etc.) peuvent être employés à cette fin. Cette homogénéisation se fait dans un tampon de composition appropriée, bien connue de l'homme du métier. L'homogénat ainsi obtenu est ensuite clarifié, le plus souvent par centrifugation, pour éliminer les grosses particules peu ou mal broyées ou encore pour obtenir la fraction cellulaire contenant la protéine recherchée. Si la protéine est justement dans un compartiment cellulaire, on utilise généralement un détergent doux (Triton, Tween, etc., quelquefois déoxycholate) pour la libérer en dissolvant les membranes de ce compartiment. L'emploi de détergent doit souvent être fait de façon contrôlée car ils peuvent briser les lysosomes, ce qui libère des enzymes hydrolytiques (protéases, nucléases, etc.) qui peuvent attaquer et détruire les protéines ou autres molécules qu'on veut isoler. Des précautions particulières doivent être prises si on travaille avec des protéines sensibles à la dégradation ou peu nombreuses. Une solution fréquente à ce problème est l'inclusion dans les solutions d'inhibiteurs de protéases qui sont soit physiologiques (inhibiteur de trypsine, antipaïne. leupeptine...) ou artificiels (E64, PMSF...). Ensuite diverses techniques existent pour isoler la protéine recherchée. Une des méthodes se prêtant le mieux à de gros volumes est la précipitation différentielle au sulfate d'ammonium. Les chromatographies d'échange ionique ou les chromatographies d'affinité, applicables à de bons volumes d'échantillon mais ayant un assez bon pouvoir de séparation, constituent de bonnes méthodes intermédiaires. Pour finaliser la purification, le tamisage moléculaire ou l'isofocalisation sont souvent utilisés. Ces techniques permettent de raffiner la pureté, mais nécessitent de très petits volumes de protéines concentrées. Il est souvent avantageux, entre ces étapes, d'éliminer les sels ou produits utilisés dans ces chromatographies. Ceci peut être obtenu par dialyse ou par ultrafiltration.

Il peut être avantageux d'utiliser un vecteur selon l'invention portant une séquence permettant d'identifier les fragments polypeptidiques de l'invention. De plus, il peut être avantageux de faciliter la sécrétion dans un système procaryote ou eucaryote. En effet, dans ce cas, les fragments recombinants d'intérêt seront présents dans le surnageant de la culture cellulaire plutôt qu'à l'intérieur des cellules hôtes.

Ainsi, en utilisant l'une ou l'autre de ces techniques, et contrairement à la forme entière de l'IL-33 humaine, les fragments polypeptidiques de l'IL-33 identifiés par les présents Inventeurs (IL-33ₐₐ₉₅₋₂₇₀, IL-33ₐₐ₉₉₋₂₇₀ et IL-33ₐₐ₁₀₉₋₂₇₀) peuvent être produits par les cellules recombinantes de l'invention en de grandes quantités (cf. exemple c) ci-dessous). Plus précisément, les fragments de l'invention peuvent être produits à des taux atteignant au moins 1mg par litre, de préférence 2 mg par litre, de manière encore plus préférée, 5mg par litre de culture cellulaire.

De manière alternative, il est possible de préparer les fragments polypeptidiques selon l'invention par synthèse chimique. Un tel procédé de préparation est également un objet de l'invention. L'homme du métier connaît les procédés de synthèse chimique, par exemple les techniques mettant en oeuvre des phases solides ou des techniques utilisant des phases solides partielles, par condensation de fragments ou par une synthèse en solution classique. Les fragments de l'invention peuvent par exemple être synthétisés par les techniques de la chimie de synthèse, telles que la synthèse de type Merrifield qui est avantageuse pour des raisons de pureté, de spécificité antigénique, d'absence de produits secondaires non désirés et pour sa facilité de production. Cette synthèse chimique peut être couplée à une approche de génie génétique ou par génie génétique seul en utilisant les techniques bien connues de l'homme du métier et décrites par exemple dans Sambrook J. et a]., Molecular Cloning : A Laboratory Manual, 1989. Les réactifs et produits de départ sont commercialement disponibles, ou peuvent être synthétisés par des techniques conventionnelles bien connues (voir par exemple, WO 00/12508, WO 2005/085260).

Les nouveaux fragments de l'IL-33 identifiés par les présents inventeurs présentent *in vitro* et *in vivo* une activité biologique supérieure à la forme entière de l'IL-33 humaine et/ou à la forme tronquée IL-33ₐₐ₁₁₂₋₂₇₀ utilisée jusqu'ici.

Ces fragments peuvent être avantageusement utilisés en tant qu'agonistes *in vitro* des récepteurs ST2 ou de tout autre récepteur se liant à l'IL-33 humaine. Dans un autre aspect, l'invention vise également les fragments de l'invention pour leur utilisation en tant qu'agonistes *in vivo* des récepteurs ST2 ou de tout autre récepteur se liant à l'IL-33 humaine. Par « récepteur ST2 » on entend de préférence la protéine de séquence SEQ ID NO : 12 (isoforme 1, NP_057316.3) ou SEQ ID NO : 13 (isoforme 2, NP_003847.2), autrement appelée « T1 », « DER4 », « IL1R1 », « FIT-1 », « MGC32623 » ou encore « IL33R ».

Les fragments de l'invention sont capables de stimuler efficacement la sécrétion d'IL-6 par des cellules mastocytaires *in vitro*, et de recruter *in vivo* les cellules de système immunitaire (augmentation du poids de la rate et du nombre de granulocytes dans le sang des souris). Elles peuvent donc avoir *in vivo* une activité thérapeutique significative, notamment contre les maladies réagissant positivement à l'administration de l'IL-33 humaine native.

Dans un autre aspect, la présente invention vise donc une composition pharmaceutique comprenant l'un au moins des fragments polypeptidiques isolés de l'invention, ou l'un au moins des acides nucléiques codant les fragments polypeptidiques de l'invention. Dans le cas où la composition comprend le fragment polypeptidique de l'invention, ledit fragment peut être obtenu par voie recombinante, ou encore par voie de synthèse chimique.

De préférence, ladite composition sera additionnée d'un excipient et/ou d'un véhicule pharmaceutiquement acceptable. Dans la présente description, on entend désigner par véhicule pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration du ou des composés actifs choisis.

De préférence, ces composés seront administrés par voie systémique, en particulier par voie intraveineuse, par voie intramusculaire, intradermique, intrapéritonéale ou sous-cutanée, par voie orale, ou par voie topique (au moyen de gel, aérosols, gouttes, etc.). De manière plus préférée, la composition pharmaceutique de l'invention sera administrée à plusieurs reprises, de manière étalée dans le temps. Son mode d'administration, sa posologie et sa forme galénique optimale peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Dans un autre aspect, l'invention concerne également les fragments polypeptidiques de l'invention, pour leur utilisation en tant que médicament.

Plus précisément, l'invention vise chacun des fragments polypeptidiques de l'invention, pour leur utilisation en tant que médicament permettant de traiter les maladies qui réagissent positivement à l'administration de l'IL-33 humaine native, comme par exemple les maladies infectieuses comme le sepsis, les kératites dues à Pseudomonas, la tuberculose ou les infections dues à un nématode ou au virus de l'influenza, l'athérosclérose, les maladies cardio-vasculaires telles que l'infarctus du myocarde, le cancer ou bien encore l'inflammation des tissus adipeux en cas d'obésité. En d'autres termes, l'invention concerne l'utilisation d'au moins un des fragments polypeptidiques de l'invention, pour la fabrication d'un médicament destiné à traiter les maladies infectieuses comme le sepsis, les kératites dues à Pseudomonas, la tuberculose ou les infections dues à un nématode ou au virus de l'influenza, l'athérosclérose, les maladies cardio-vasculaires telles que l'infarctus du myocarde, le cancer ou bien encore l'inflammation des tissus adipeux en cas d'obésité.

Les maladies cardiovasculaires visées par la présente invention sont par exemple les maladies des coronaires, les maladies du muscle cardiaque, les maladies des valves cardiaques, la maladie du péricarde, les maladies du rythme ou de la conduction cardiaque, les maladies des vaisseaux ou autres. Les maladies des coronaires visées sont par exemple l'angine de poitrine ou l'infarctus du myocarde. Les maladies du muscle cardiaque visées par l'invention sont par exemple la cardiomyopathie ou l'insuffisance cardiaque. Les maladies des valves cardiaques visées sont par exemple l'endocardite, ou les valvulopathies cardiaques. Par « maladie du péricarde », on entend communément la péricardite. Les maladies du rythme ou de la conduction cardiaque sont par exemple la syncope d'origine cardiovasculaire, les troubles de la conduction cardiaque, ou les troubles du rythme cardiaque. Les maladies des vaisseaux visées sont par exemple l'anévrisme, l'artériopathie oblitérante des membres inférieurs, la dissection aortique aiguë, l'hypertension artérielle pulmonaire, ou la maladie thrombo-embolique. Les autres maladies visées par l'invention sont par exemple l'arrêt cardio-circulatoire, la cardiopathie congénitale, l'hypertension artérielle, l'hypotension artérielle, ou le syndrome d'Adams-Stokes.

Parmi les cancers qui peuvent être prévenus et/ou traités par le(s) fragment(s) polypeptidique(s) de l'invention, sont particulièrement visés le cancer de la prostate, les ostéosarcomes, le cancer du poumon, le cancer du sein, le cancer de l'endomètre, le cancer du côlon, le myélome multiple ou le cancer des ovaires, le cancer du pancréas, le mélanome ou tout autre cancer.

La présente invention vise également une méthode de traitement comprenant l'administration à un patient d'une composition pharmaceutique comprenant une quantité efficace de l'un des fragments polypeptidiques de l'invention ou un des acides nucléiques de l'invention codant pour celui-ci. De préférence cette méthode de traitement permet de prévenir et ou traiter les maladies infectieuses comme le sepsis, la kératite dues à *Pseudomonas,* la tuberculose ou les infections dues à un nématode ou au virus de l'influenza, l'athérosclérose, les maladies cardio-vasculaires, le cancer, ou bien encore l'inflammation des tissus adipeux en cas d'obésité, chez les patients qui en souffrent.

La présente invention vise donc en particulier une composition pharmaceutique comprenant l'acide nucléique de l'invention ou le vecteur nucléotidique de l'invention. Cette composition pharmaceutique peut donc être utilisée à des fins de thérapie génique.

A cette fin, la présente invention vise également les cellules de mammifères, y compris les cellules humaines, de préférence non-embryonnaires, comprenant l'un des vecteurs ou l'un des acides nucléiques de l'invention. Dans le cadre de la présente invention, le principe de la thérapie génique est d'administrer à un patient un acide nucléique qui code le ou les fragments polypeptidiques d'intérêt de l'invention, dans des conditions telles qu'ils sont exprimés *in vivo* par les cellules du patient dans lesquelles l'acide nucléique a été transféré.

Un tel acide nucléique peut être notamment sous la forme d'un vecteur d'ADN, par exemple un vecteur plasmidique. On peut administrer un ou plusieurs vecteurs, chaque vecteur pouvant porter une ou plusieurs séquence (s) codant pour au moins un des fragments polypeptidiques d'intérêt. Le ou les vecteurs d'ADN peuvent être introduits *in vivo* par toute technique connue de l'homme du métier. En particulier, il est possible d'introduire le vecteur d'ADN *in vivo* sous une forme nue, c'est-à-dire sans l'aide d'un quelconque véhicule ou système qui faciliterait la transfection du vecteur dans les cellules (EP 465 529). Un « canon à gènes » peut être également employé, par exemple en déposant l'ADN à la surface de particules « en or » et en projetant celles-ci de manière à ce que l'ADN pénètre à travers la peau d'un patient. Des injections au moyen d'un gel liquide sont également possibles pour transfecter à la fois peau, muscle, tissu gras et tissu mammaire (Furth et al., Anal Biochem 1992). Des techniques de microinjection, électroporation, précipitation au phosphate de calcium, formulations à l'aide de nanocapsules ou de liposomes sont d'autres techniques disponibles. Des nanoparticules en polyalkyl cyanoacrylate biodégradables sont particulièrement avantageuses. Dans le cas de liposomes, l'utilisation de lipides cationiques favorise l'encapsulation des acides nucléiques qui sont chargés négativement, et facilite la fusion avec les membranes cellulaires chargées négativement. De manière alternative, le vecteur peut être sous la forme d'un virus recombinant comprenant, insérée dans son génome, une séquence d'acide nucléique qui code pour le ou lesdits peptide (s). Le vecteur viral peut être de préférence choisi parmi un adénovirus, un rétrovirus, en particulier un lentivirus, ainsi qu'un virus adéno-associé (AAV), un virus de l'herpès, un cytomégalovirus (CMV), un virus de la vaccine, etc. De manière avantageuse, le virus recombinant est un virus défectif.

Le terme « virus défectif » désigne un virus incapable de se répliquer dans une cellule cible. Généralement, le génome des virus défectifs est dénué d'au moins les séquences nécessaires pour la réplication dudit virus dans la cellule infectée. Ces régions peuvent être soit éliminées, soit rendues non fonctionnelles ou encore substituées par d'autres séquences et en particulier par l'acide nucléique qui code pour le fragment polypeptidique d'intérêt. Néanmoins, de préférence, le virus défectif conserve malgré tout les séquences de son génome nécessaires à l'encapsulation des particules virales. Une administration ciblée de gènes est par exemple décrite dans la demande WO 95/28 494.

Dans un autre aspect, la présente invention vise l'utilisation d'un fragment polypeptidique selon l'invention, éventuellement obtenu par un des procédés de l'invention, ou l'acide nucléique de l'invention, en combinaison avec un autre médicament, par exemple en combinaison avec un agent chimiothérapeutique pour le traitement du cancer, associé à une prothèse vasculaire (ou stent) pour le traitement par angioplastie des maladies cardiovasculaires, ou en tant qu'adjuvant d'un vaccin contre un virus, comme le virus de l'influenza.

Dans un mode de réalisation préféré, le fragment polypeptidique de l'invention est combiné à un agent chimiothérapeutique compris dans le groupe constitué par : les agents alkylants, les antimétabolites, les alcaloïdes végétaux, les inhibiteurs de la topoisomérase, les antibiotiques antitumoraux, ou les poisons du fuseau mitotiques. Par « agents alkylants » sont visés entre autres le busulfan, la carboplatine, le chlorambucil, le cisplatine, le cyclophosphamide, l'isofamie, le melphalan, la méchloréthamine, l'oxaliplatine, l'uramustine, ou encore la témozolomide. Par « antimétabolites » sont visés entre autres l'azathioprine, la capécitabine, la cytarabine, la floxuridine, la fludarabine, le fluorouracil, la gemcitabine, le méthotrexate, ou encore le pémétrexed. Les « alcaloïdes végétaux » sont par exemple la vinblastine, et la vincristine. Les « inhibiteurs de la topoisomérase » sont par exemple l'irinotécan, le topotécan, ou encore l'étoposide. Les « antibiotiques antitumoraux » sont par exemple la bléomycine, la Daunorubicine, la doxorubicine, l'épirubicine, l'hydroxyurée, l'idarubicine, la mitomycine C, ou encore la mitoxantrone. Enfin, les « poisons du fuseau mitotique » sont par exemple le docétaxel, le paclitaxel, la vinblastine, la vincristine, ou encore la vinorelbine.

Selon un autre aspect, la présente invention vise l'utilisation d'un fragment polypeptidique isolé selon l'invention, éventuellement obtenu par un des procédés de l'invention, ou l'acide nucléique de l'invention, pour diagnostiquer une infection ou un traumatisme chez un individu.

Le diagnostic pourrait être établi en utilisant une technique ELISA permettant de détecter spécifiquement les nouveaux fragments de l'IL-33 (IL-33ₐₐ₉₅₋₂₇₀ IL-33ₐₐ₉₉₋₂₇₀ et IL-33ₐₐ₁₀₉₋₂₇₀) dans le sérum ou le plasma des patients.

En effet, une augmentation de la quantité d'IL-33 dans le sang ou le sérum pourrait témoigner d'une infection, d'une inflammation ou d'un traumatisme chez un individu. Il est donc envisageable d'utiliser les fragments polypeptidiques de l'invention, qui sont susceptibles d'êtres formés *in vivo* par l'action des protéases endogènes, comme marqueur d'un état infectieux ou inflammatoire.

Avantageusement, ces fragments particuliers d'IL-33 peuvent être détectés au moyen d'anticorps les reconnaissant spécifiquement.

Dans un ultime aspect, la présente invention vise donc un anticorps monoclonal reconnaissant spécifiquement l'un des fragments polypeptidiques de la présente invention, mais ne reconnaissant pas la forme entière IL-33ₐₐ₁₋₂₇₀ ni la forme tronquée IL-33ₐₐ₁₁₂₋₂₇₀. De façon avantageuse, ledit anticorps est un anticorps conformationnel, c'est-à-dire un anticorps reconnaissant un épitope conformationnel.

Un anticorps selon l'invention peut être préparé par les techniques classiques connues de l'Homme du métier, telles que celles décrites dans Antibodies : A Laboratory Manual, E. Howell and D Lane, Cold Spring Harbor Laboratory, 1988.

De manière plus précise, un tel anticorps peut être produit à partir d'hybridomes obtenus par fusion de lymphocytes B d'un animal immunisé par l'un ou plusieurs des fragments polypeptidiques de l'invention, avec des myélomes, selon la technique de Kôhler et Milstein (Nature, 1975) ; les hybridomes sont cultivés *in vitro*, notamment dans des fermenteurs, ou produits *in vivo,* sous forme d'ascite ; alternativement ledit anticorps monoclonal peut être produit par génie génétique comme décrit dans le brevet américain US 4,816, 567.

Les anticorps selon l'invention qui reconnaissent spécifiquement les fragments polypeptidiques de l'invention avec une affinité élevée (constante d'affinité de l'ordre du nM), représentent des réactifs de diagnostic fiables et sensibles pour la détection, à partir d'un échantillon biologique approprié (sang total, cellules mononucléées du sang périphérique, biopsie tumorale), des patients souffrant d'infections latentes ou de traumatisme.

Dans un mode de réalisation particulier, la présente invention vise donc également une méthode de diagnostique d'infections ou de traumatismes utilisant les anticorps de l'invention. Cette méthode diagnostique peut utiliser ces anticorps dans des techniques de cytométrie de flux, d'immunocytochimie ou d'immunohistochimie ou bien d'immunoprécipitation à partir de cellules non-fixées (congelées ou vivantes) ou fixées dans des conditions non-dénaturantes qui sont connues de l'homme du métier.

L'invention sera décrite plus précisément au moyen des exemples ci-dessous.

### Exemples

### a) Mise en évidence des fragments de l'IL-33 de l'invention

Les protéines IL-33 humaines entières (IL-33₁₋₂₇₀, SEQ ID NO : 1) ou tronquées (IL-33₁₁₂₋₂₇₀, SEQ ID NO : 5) ont été produites *in vitro* selon les techniques classiques de production de protéines dans des lysats de réticulocytes selon les recommandations du fabricant (Promega TnT Coupled Reticulocyte Lysate Systems ref L4610) : 1µg de plasmide permettant l'expression d'IL-33 pleine taille ou tronquée (pcDNA3-IL33 1-270 ou 95-270 ou 99-270 ou 109-270) est mélangé au lysat de réticulocyte de lapin (25µl) contenant le tampon de réaction 10X (2µl), la polymérase T7 (1µl), le mélange d'acides aminés 1mM (1µl), l'inhibiteur de RNase RNAsin (40U/µl) dans un volume réactionnel de 50µl final. Un contrôle est effectué en utilisant le vecteur vide pcDNA3. Une incubation est réalisée à 30°C pendant 90min. Pour le clivage par les protéases des neutrophiles, 5µl de lysat programmé ou non sont ensuite incubés à 37°C pendant 1h avec 1mU de cathepsine G (Calbiochem, ref: 219373), ou 30 min avec 0.3U d'élastase (Calbiochem ref: 324681) ou 30 min avec 0.25µg de PR3 (Calbiochem ref: 539483). Les produits de clivage sont analysés par gel SDS-PAGE et Western Blot à l'aide d'un anticorps monoclonal dirigé contre la partie C-terminale de l'IL-33 (IL-33305B, Alexis Biochemical ref: ALX-804-726) (figure 2A). L'IL-33 humaine est la cible des protéases recombinantes Cathepsine G (Cat G), Elastase 2 (Elastase), et Protéinase 3 (PR3), qui génèrent *in vitro* les trois fragments matures IL-33ₐₐ₉₅₋₂₇₀, IL-33ₐₐ₉₉₋₂₇₀ et IL-33ₐₐ₁₀₉₋₂₇₀. En revanche, les trois protéases de neutrophiles ne clivent pas le domaine de type cytokine IL-1 (domaine C-terminal), comme le montrent les deux blots du dessous sur la figure 2A.

En outre, des neutrophiles humains ont été isolés et purifiés à partir de sang frais total humain collecté sur tube EDTA, en utilisant le milieu de séparation Polymorphprep (Axis-Shield ; ref 1114683). Les neutrophiles sont resuspendus dans du RPMI/Hepes (25mM) et répartis dans des plaques de 96 puits à raison de 100.000 neutrophiles/ml et 100µl/puits, puis activés à l'aide de PMA (Acétate du myristate 13 du Phorbol 12, 25nM) pendant 2 heures à 37°C. La protéine IL-33 entière (25µl de lysat programmé comme décrit ci-dessus) a été incubée avec ces neutrophiles humains, en présence ou en absence d'inhibiteur de protéases à sérine (AEBSF, 5mM). L'analyse des fragments générés a été réalisée par Western-Blot à l'aide d'un anticorps anti-IL-33 dirigé contre la partie C-terminale (IL-33305B ; Alexis Biochemical, ref: ALX-804-726). Une comparaison des tailles des fragments générés a été réalisée à l'aide des protéines recombinantes IL-33ₐₐ₉₅₋₂₇₀, IL-33ₐₐ₉₉₋₂₇₀ et IL-33ₐₐ₁₀₉₋₂₇₀. Les résultats obtenus sont présentés sur la figure 2B. Ils permettent de démontrer que l'IL-33 humaine est effectivement la cible *ex vivo* des protéases de neutrophiles humains Cathepsine G (Cat G), Elastase 2 (Elastase), et Protéinase 3 (PR3).

### b) Les nouveaux fragments de l'IL-33 sont 'superactifs'in vitro.

L'activité biologique des nouveaux fragments de l'IL-33, IL-33ₐₐ₉₅₋₂₇₀, IL-33ₐₐ₉₉₋₂₇₀ et IL-33ₐₐ₁₀₉₋₂₇₀, a été testée *in vitro* dans deux essais fonctionnels, l'un basé sur la sécrétion de l'IL-5 dépendante de ST2 dans une lignée de basophile humain, et l'autre basé sur la sécrétion d'IL-6 dépendante de ST2 dans une lignée mastocytaire murine.

Brièvement, les cellules mastocytaires MC/9 (ATCC CRL-8306) sont maintenues dans du milieu DMEM (ATCC, Ref: 30-2002) contenant 6mM de glutamine, 0.05mM de 2-mercaptoéthanol, 10% de Rat-T-STIM (Beckton-Dickinson, ref: 354115) et 10% de sérum de veau foetal (ATCC, ref : 30-2020). 200.000 cellules par puits sont réparties dans des plaques de 96 puits à raison de 200µl / puits puis incubées pendant 24 heures à 37°C avec les protéines IL-33ₐₐ₁₋₂₇₀, IL-33ₐₐ₉₅₋₂₇₀, IL-33ₐₐ₉₉₋₂₇₀ et IL-33ₐₐ₁₀₉₋₂₇₀ (10µl de lysat de réticulocyte programmé). La quantité d'IL-6 sécrétée dans le surnageant de culture a été mesurée de façon classique grâce à la technique ELISA à l'aide du kit 'mouse IL-6' (R&D ref: DY406). Les résultats ont été ramenés à la valeur obtenue pour l'IL-33 pleine taille IL-33ₐₐ₁₋₂₇₀.

Les cellules de basophiles humaines KU812 (ATCC CRL-2099) sont maintenues dans du milieu RPMI 1640 (ATCC, Ref: 30-2001) supplémenté de 10% de sérum de veau foetal (ATCC, ref: 30-2020). 10⁶ cellules par puits sont réparties dans des plaques de 96 puits à raison de 200µl/ puits, puis incubées pendant 24 heures à 37°C avec les protéines IL-33ₐₐ₁₋₂₇₀, IL-33ₐₐ₉₅₋₂₇₀, IL-33ₐₐ₉₉₋₂₇₀ et IL-33ₐₐ₁₀₉₋₂₇₀ (8µl de lysat de réticulocyte programmé). La quantité d'IL-5 sécrétée dans le surnageant de culture a été mesurée de façon classique grâce à la technique ELISA à l'aide du kit 'human IL-5' (R&D, ref: DY205).

Dans chacun de ces essais, il a été mis en évidence que les protéines recombinantes IL-33ₐₐ₉₅₋₂₇₀, IL-33ₐₐ₉₉₋₂₇₀ et IL-33ₐₐ₁₀₉₋₂₇₀, présentaient une activité plus grande que celle de la forme active pleine taille IL-33ₐₐ₁₋₂₇₀ (Figure 3B et 3C). Les nouveaux fragments de l'IL-33, IL-33ₐₐ₉₅₋₂₇₀, IL-33ₐₐ₉₉₋₂₇₀ et IL-33ₐₐ₁₀₉₋₂₇₀, sont donc 'superactifs' par rapport à la forme entière *in vitro*.

### c) Procédé de production/purification des fragments d'IL-33 de l'invention par voie recombinante.

Les protéines recombinantes IL-33 humaines entière ou tronquées ont été produites chez la bactérie en fusion avec une étiquette 6xHistidine en N-terminal pour permettre la purification des protéines, selon les techniques classiques utilisées par l'homme de l'art. Le Tag Histidine a été ensuite éliminée par un clivage à la thrombine et la protéine d'intérêt a été purifiée par chromatographie d'exclusion stérique.

Brièvement, un litre de milieu de culture est ensemencé avec la souche transformée (BL21 pLysS-pET15b-IL-33ₐₐ₁₋₂₇₀ ou BL21 pLysS-pET15b-IL-33ₐₐ₉₅₋₂₇₀, ou BL21 pLysS-pET15b-IL-33ₐₐ₉₉₋₂₇₀ ou BL21 pLysS-pET15b-IL-33ₐₐ₁₀₉₋₂₇₀). Lorsque la DO atteint 0,7, l'expression de la protéine d'intérêt est induite par l'ajout de 1mM d'IPTG pendant 4 heures à 37 °C. Les bactéries sont centrifugées et le culot est repris dans du tampon de lyse (Na₂HPO₄ 50mM, NaCl 300mM, Imidazole 20mM, Triton 0.5%, Dnase I 1mM, lyzosyme 1mM, inhibiteur de protéases). Après sonication et ultracentrifugation, le lysat bactérien est récupéré et incubé avec des billes Nickel-NTA à 4° pendant 4 heures. Les billes sont lavées et la protéine purifiée par affinité est éluée avec 500 mM d'imidazole. L'éluat est ensuite débarrassé de l'imidazole et dialysé en tampon Tris-Nacl par centrifugation/concentration à l'aide de Vivaspin (Sartorius ; 20mL de seuil 10kDa). Le tag histidine est clivé par la thrombine (250 unités/mL, 4°C, 20h) et la protéine est ensuite purifiée par chromatographie sur gel perméable ('Gel Filtration' - colonne Superdex 75) suivie d'une concentration par Vivaspin (Sartorius). L'analyse des différentes étapes de purification est effectuée par gel SDS-PAGE et coloration au bleu de Coomassie. Les protéines purifiées sont quantifiées par dosage au Nanodrop (Thermoscientific), et la présence d'endotoxine a été déterminée en utilisant le kit 'Limulus Amebocyte Lysate' (Cambrex, Ref: 50-647U).

Des exemples de production/purification des protéines IL-33 (formes entière ou clivées) sont montrés dans la figure 4. Au moins 9 mg de protéines correspondantes aux fragments d'IL-33 (IL-33₉₅₋₂₇₀, IL-33₉₉₋₂₇₀, IL-33₁₀₉₋₂₇₀) ont pu être produites à partir d'un litre de culture bactérienne, tandis que moins d'1 µg a pu être obtenu pour la forme pleine taille. Le rendement pour la production des fragments d'IL-33 est donc au moins 9000 fois supérieur à celui obtenu pour la forme pleine taille 1-270.

### d) Les nouveaux fragments de l'IL-33 sont biologiquement 'superactifs' in vivo

L'activité biologique des nouveaux fragments de l'IL-33 a été testée *in vivo* chez la souris et comparée à l'activité de la forme artificielle IL-33ₐₐ₁₁₂₋₂₇₀. L'utilisation de la protéine humaine IL-33 entière dans cette expérience est exclue puisqu'il est impossible d'obtenir la quantité requise de protéine pour l'injection (>340µg, en tenant compte du nombre de souris à injecter, et du nombre total d'injections à effectuer). Les protéines recombinantes IL-33ₐₐ₁₁₂₋₂₇₀, IL-33ₐₐ₉₅₋₂₇₀, IL-33ₐₐ₉₉₋₂₇₀ et IL-33ₐₐ₁₀₉₋₂₇₀ produites chez *Escherichia coli* (vecteur pET15b, Novagen ; bactérie BL21) et purifiées par chromatographie d'affinité ont été injectées par voie intra-péritonéale (4 µg dans 200µl PBS) dans des souris Balb/C (12 souris femelles de 8 à 10 semaines) pendant 7 jours, quotidiennement. L'analyse des souris a montré des changements pathologiques nets, avec notamment une augmentation du volume et poids de la rate au bout de 7 jours (figure 4A), et une hyperplasie des cellules caliciformes du jejunum (figure 4B). De façon intéressante, deux des nouveaux fragments de l'IL-33 (IL-33ₐₐ₉₅₋₂₇₀ et IL-33ₐₐ₉₉₋₂₇₀) induisent une augmentation du nombre de granulocytes (majoritairement des neutrophiles) dans le sang des souris après 7 jours, augmentation significativement plus élevée que celle induite par la forme tronquée IL-33ₐₐ₁₁₂₋₂₇₀ (Figure 4C), indiquant que les nouveaux fragments de l'IL-33, IL-33ₐₐ₉₅₋₂₇₀ et IL-33ₐₐ₉₉₋₂₇₀, sont 'superactifs' *in vivo.* Ce dernier résultat est tout à fait inattendu car l'existence de fragments de l'IL-33 plus actifs que la forme IL-33ₐₐ₁₁₂₋₂₇₀ n'avait jamais été suspectée auparavant.

### Références bibliographiques

Adra et al., 1987, Gene, 60 : 65-74
Ali et al., Biochem Biophys Res Commun 2010, 391 :1512-6
Alves-Filho et al., Nature Medicine 2010, 16 :708-12
Baekkevold E. et al., American Journal Pathology 2003, 163 :69-79
Bieth JG, J. Soc. Biol. 2001, 195(2): 173-9
Buckholz RG, Current Opinion in Biotechnology 1993, 4(5):538-42
Burster T. et al; Molecular Immunology 2010, 47:658-665
Carrière V. et al., PNAS 2007, 104 :282-287
Cayrol and Girard, PNAS 2009, 106(22) :9021-6
Chackerian AAet al., Journal of Immunology 2007, 179: 2551-5
Ciernik I. F., et al., The Journal of Immunology, 1999, 162, 3915-3925
David L. Nelson and Michael M. Cox, Lehninger Principes of Biochemistry, 3rd edition, 2000
Edwards CP et Aruffo A, Current Opinion in Biotechnology 1993, 4(5):558-63
Furth PA et al., 1992 Anal Biochem. 1992 Sep;205(2):365-8
Hausman RE et al (2004). The cell: a molecular approach. Washington, D.C: ASM Press. p. 51
Hajjar E. et al., FEBS Letters 2007, 581 :5685-5690.
Hazlett et al., Invest Ophtalmol Vis Sci 2010, 51 :1524-32
Humphreys et al., Journal of Immunology 2008, 180 :2443-9
Kargi et al, 1990, J. Histochem. Cytochem.38(8) : :1179-86
Kôhler et Milstein, Nature, 1975,256, 495-497.
Korkmaz B. et al., Pharmacological Reviews 2010, 62 : 726-759.
Liew et al., Nature Rev Immunology 2010, 10 :103-110
Lingel et al., Structure 2009, 17 :1398-410
Luckow VA, Journal of Virology 1993, 67(8) 4566-79
Lüthi et al., Immunity 2009 ; 31 :84-98
Miller et al., J. Exp. Med 2008, 205 :339-46
Miller et al. Circ Res 2010 107:650-8
Moessler et al., Development1996, 122 : 2415- 2425.
Moussion et al., PLoS ONE 2008, 3(10) :e3331
Nufer O et al., Biochemistry 1999, 38(2):636-42
Olins et Lee, Current Opinion in Biotechnology1993, 4(5) :520-5
Pushparaj et al., PNAS 2008, 106 :9773-8
Rankin et al., J Immunol 2010, 184:1526-35.
Rao NV et al, PNAS 1991, 88; 9253-9256
Reen DJ, Methods Mol Biol. 1994, 32:461-6.
Sambrano G.R. et al., J. Biol Chem. 2000, 275 : 6819-6823.
Schmitz et al., Immunity 2005, 23 :479-90
Seki et al., Circ Heart Fail 2009, 2 :684-91
Sims JE and Smith DE, Nature Rev Immunology 2010, 10:89-102
Stolarski et al., Journal of Immunology 2010, 185 :3472-80
Talabot-Ayer et al., J. Biol. Chem 2009 ; 284 :19420-6
Tatusova et al, FEMS Microbiol., 1999 Lett. 174:247-250
Xu et al., Journal of Immunology 2010; 184 :2620-6

## Revendications

1. Fragment polypeptidique isolé de l'interleukine 33 humaine (hIL-33), biologiquement actif, choisi parmi les fragments ayant pour séquence les acides aminés 95 à 270 (SEQ ID NO :2), 99 à 270 (SEQ ID NO :3) ou 109 à 270 (SEQ ID NO :4) de l'interleukine 33 humaine (hIL-33), lesdits fragments étant des produits naturels de clivage de hIL-33 par au moins une protéase des neutrophiles humains.

2. Fragment polypeptidique isolé selon la revendication 1, **caractérisé en ce que** ladite protéase des neutrophiles humains est choisie parmi: la cathepsine G (EC 3.4.21.20), la protéinase 3 des leucocytes (EC 3.4.21.76), et l'élastase 2 des neutrophiles (EC 3.4.21.37).

3. Fragment polypeptidique isolé selon l'une quelconque des revendications 1 ou 2, **caractérisé en ce qu'**il présente au moins une activité biologique améliorée par rapport à l'interleukine 33 humaine (hIL-33).

4. Fragment polypeptidique isolé selon l'une quelconque des revendications 1 à 3, **caractérisé en ce qu'**il présente au moins une activité biologique améliorée par rapport à la forme tronquée ayant pour séquence les acides aminés 112 à 270 de l'hIL-33 (SEQ ID NO :5).

5. Acide nucléique isolé codant un fragment polypeptidique selon l'une quelconque des revendications 1 à 4.

6. Vecteur nucléotidique **caractérisé en ce qu'**il comprend un acide nucléique selon la revendication 5.

7. Cellule hôte **caractérisée en ce qu'**elle est transformée par un acide nucléique selon la revendication 5 ou le vecteur selon la revendication 6.

8. Fragment polypeptidique tel que défini dans l'une quelconque des revendications 1 à 4, pour son utilisation en tant qu'agoniste du récepteur ST2.

9. Fragment polypeptidique tel que défini dans l'une quelconque des revendications 1 à 4, pour son utilisation en tant que médicament.

10. Fragment polypeptidique tel que défini dans l'une quelconque des revendications 1 à 9, pour son utilisation dans le traitement des maladies infectieuses comme le sepsis, les kératites dues à Pseudomonas ou la tuberculose, des infections dues à un nématode ou au virus de l'influenza, de l'athérosclérose, des maladies cardio-vasculaires telles que l'infarctus du myocarde, du cancer ou de l'inflammation des tissus adipeux en cas d'obésité.

11. Composition pharmaceutique comprenant au moins un fragment polypeptidique isolé selon l'une quelconque des revendications 1 à 4, ou l'acide nucléique défini à la revendication 5.

12. Utilisation d'un fragment polypeptidique isolé selon l'une quelconque des revendications 1 à 4, ou de l'acide nucléique défini à la revendication 5, pour diagnostiquer une infection ou un traumatisme chez un individu.

13. Anticorps monoclonal reconnaissant spécifiquement le fragment isolé tel que défini dans l'une quelconque des revendications 1 à 4.

14. Procédé de production d'un fragment polypeptidique isolé tel que défini dans les revendications 1 à 4, **caractérisé en ce qu'**il comprend les étapes suivantes :
a) culture d'une cellule recombinante telle que définie à la revendication 7, dans un milieu de culture et des conditions de culture appropriées,
b) isolement du fragment polypeptidique obtenu à l'étape a).

## Patentansprüche

1. Isoliertes Polypeptidfragment vom biologisch aktiven menschlichen Interleukin 33 (hIL-33), ausgewählt aus den Fragmenten, die als Sequenz die Aminosäuren 95 bis 270 (SEQ ID Nr.: 2), 99 bis 270 (SEQ ID Nr.: 3) oder 109 bis 270 (SEQ ID Nr.: 4) des menschlichen Interleukins 33 (hIL-33) aufweisen, wobei die Fragmente natürliche Spaltungsprodukte von hIL-33 durch mindestens eine Protease der menschlichen Neutrophile sind.

2. Isoliertes Polypeptidfragment nach Anspruch 1, **dadurch gekennzeichnet, dass** die Protease der menschlichen Neutrophile ausgewählt ist aus: Cathepsin G (EC 3.4.21.20), Proteinase 3 der Leukozyten (EC 3.4.21.76) und Elastase 2 der Neutrophilen (EC 3.4.21.37).

3. Isoliertes Polypeptidfragment nach einem der Ansprüche 1 oder 2, **dadurch gekennzeichnet, dass** es mindestens eine biologische Aktivität aufweist, die mit Bezug auf das menschliche Interleukin 33 (hIL-33) verbessert ist.

4. Isoliertes Polypeptidfragment nach einem der Ansprüche 1 bis 3, **dadurch gekennzeichnet, dass** es mindestens eine biologische Aktivität aufweist, die mit Bezug auf die verkürzte Form als Sequenz die Aminosäuren 112 bis 270 von hIL-33 (SEQ ID Nr.: 5) aufweist.

5. Isolierte Nukleinsäure, die ein Polypeptidfragment nach einem der Ansprüche 1 bis 4 codiert.

6. Nukleotidvektor, **dadurch gekennzeichnet, dass** er eine Nukleinsäure nach Anspruch 5 umfasst.

7. Wirtszelle, **dadurch gekennzeichnet, dass** sie durch eine Nukleinsäure nach Anspruch 5 oder den Vektor nach Anspruch 6 umgewandelt wird.

8. Polypeptidfragment, wie in einem der Ansprüche 1 bis 4 definiert, für seine Verwendung als Agonist des Rezeptors ST2.

9. Polypeptidfragment, wie in einem der Ansprüche 1 bis 4 definiert, für seine Verwendung als Arzneimittel.

10. Polypeptidfragment, wie in einem der Ansprüche 1 bis 9 definiert, für seine Verwendung bei der Behandlung von Infektionskrankheiten wie z.B. Sepsis, Keratitis aufgrund von Pseudomonas oder Tuberkulose, Infektionen aufgrund einer Nematode oder des Influenzavirus, Atherosklerose, Herz-Kreislauferkrankungen wie z.B. Herzinfarkt, Krebs oder Entzündung der Fettgewebe im Fall von Fettleibigkeit.

11. Pharmazeutische Zusammensetzung, umfassend mindestens ein isoliertes Polypeptidfragment nach einem der Ansprüche 1 bis 4 oder die Nukleinsäure, wie in Anspruch 5 definiert.

12. Verwendung eines isolierten Polypeptidfragments nach einem der Ansprüche 1 bis 4 oder der Nukleinsäure, wie in Anspruch 5 definiert, um eine Infektion oder ein Trauma bei einem Patienten zu diagnostizieren.

13. Monoklonaler Antikörper, der spezifisch das isolierte Fragment, wie in einem der Ansprüche 1 bis 4 definiert, erkennt.

14. Verfahren zur Herstellung eines isolierten Polypeptidfragments, wie in den Ansprüchen 1 bis 4 definiert, **dadurch gekennzeichnet, dass** es die folgenden Schritte umfasst:
a) Kultivieren einer rekombinanten Zelle, wie in Anspruch 7 definiert, in einem Kulturmedium und geeigneten Kulturbedingungen,
b) Isolieren des Polypeptidfragments, das in Schritt a) erhalten wurde.

## Claims

1. Polypeptide fragment isolated from human interleukin 33 (hIL-33), biologically active, chosen from the fragments having as their sequence the amino acids 95 to 270 (SEQ ID NO: 2), 99 to 270 (SEQ ID NO: 3) or 109 to 270 (SEQ ID NO: 4) of human interleukin 33 (hIL-33), said fragments being natural products of the cleaving of hIL-33 by at least one protease of human neutrophils.

2. Isolated polypeptide fragment according to claim 1, **characterised in that** said protease of human neutrophils is chosen from: cathepsin G (EC 3.4.21.20), leukocyte proteinase 3 (EC 3.4.21.76), and neutrophil elastase 2 (EC 3.4.21.37).

3. Isolated polypeptide fragment according to either one of claims 1 or 2, **characterised in that** it has at least one biological activity improved with respect to human interleukin 33 (hIL-33).

4. Isolated polypeptide fragment according to any one of claims 1 to 3, **characterised in that** it has at least one improved biological activity with respect to the truncated form having as its sequence amino acids 112 to 270 of hIL-33 (SEQ ID NO: 5).

5. Isolated nucleic acid coding a polypeptide fragment according to any one of claims 1 to 4.

6. Nucleotide vector, **characterised in that** it comprises a nucleic acid according to claim 5.

7. Host cell, **characterised in that** it is transformed by a nucleic acid according to claim 5 or the vector according to claim 6.

8. Polypeptide fragment as defined in any one of claims 1 to 4, for use thereof as agonist of the receptor ST2.

9. Polypeptide fragment as defined in any one of claims 1 to 4, for use thereof as a medication.

10. Polypeptide fragment as defined in any one of claims 1 to 9, for use thereof in the treatment of infectious illnesses such as sepsis, keratitis due to *Pseudomonas* or tuberculosis, infections due to a nematode or to the influenza virus, atherosclerosis, cardiovascular ailments such as myocardial infarction, cancer or inflammation of the adipose tissues in the case of obesity.

11. Pharmaceutical composition comprising at least one isolated polypeptide fragment according to any one of claims 1 to 4, or the nucleic acid defined in claim 5.

12. Use of an isolated polypeptide fragment according to any one of claims 1 to 4, or of the nucleic acid defined in claim 5, for diagnosing an infection or trauma in an individual.

13. Monoclonal antibodies specifically recognising the isolated fragment as defined in any one of claims 1 to 4.

14. Method for producing an isolated polypeptide fragment as defined in claims 1 to 4, **characterised in that** it comprises the following steps:
a) culture of a recombinant cell as defined in claim 7, in a culture medium and under suitable culture conditions,
b) isolation of the polypeptide fragment obtained at step a).
